# EUROPEAN PATENT APPLICATION

(11) **EP 2 434 011 A1**
(43) Date of publication of application: **28.03.2012**
(21) Application number: 10777752.6
(22) Date of filing: 18.05.2010
(51) Int. Cl.: C12N 15/09, C12N 1/15, C12N 1/19, C12N 1/21, C12N 5/10, C12N 9/10, C12P 19/56

(54) **GLUCOSYLTRANSFERASE SPECIFIC TO POSITION-4 OF FUROFURAN-TYPE LIGNAN, AND POLYNUCLEOTIDE ENCODING SAME**

(30) Priority: 19.05.2009 JP 2009120637
(71) Applicant: Suntory Holdings Limited, Kita-ku, Osaka-shi Osaka 530-8203 (JP)
(72) Inventor: ONO Eiichiro, Mishima-gun Osaka 618-8503 (JP); SATAKE Honoo, Mishima-gun Osaka 618-8503 (JP); KIM Hyun-Jung, Mishima-gun Osaka 618-8503 (JP)
(74) Representative: Vossius & Partner
(86) International application number: PCT/JP2010/058352
(87) International publication number: WO 2010/134519

(57) **Abstract**

The invention provides a polynucleotide encoding a furofuran lignan 4-O-glucosyltransferase, and others. The polynucleotide in accordance with the present invention comprises a polynucleotide consisting of the nucleotide sequence represented by SEQ ID NO: 3 or SEQ ID NO: 5, or a polynucleotide encoding a protein consisting of the amino acid sequence represented by SEQ ID NO: 4 or SEQ ID NO: 6.

### [Sequence Listing]

SEQUENCE LISTING

<110>  Suntory Holdings Limited

<120>  Glucosyltransferase specific to position-4 of furofuran-type
       lignan, and polynucleotide encoding same

<130>  T3246 EP S3

<140>  EP 10 77 7752.6
<141>  2010-05-18

<150>  JP 2009-120637
<151>  2009-05-19

<160>  14

<170>  PatentIn version 3.4

<210>  1
<211>  24
<212>  DNA
<213>  Artificial

<220>
<223>  Synthetic DNA

<400>  1
atggggaagc aagaagatgc agag                                              24


<210>  2
<211>  23
<212>  DNA
<213>  Artificial

<220>
<223>  Synthetic DNA

<400>  2
ctacttactt atagaaacgc cgt                                               23


<210>  3
<211>  1407
<212>  DNA
<213>  Forsythia koreana


<220>
<221>  CDS
<222>  (1)..(1407)

<400>  3
atg gca gaa aca aag aaa tca gag ctt gtt ttc att cct gtt cca ggt       48
Met Ala Glu Thr Lys Lys Ser Glu Leu Val Phe Ile Pro Val Pro Gly
1               5                   10                  15

gta ggt cac ttg ata tca act att gag atg gct gag ctt ctc act gat       96
Val Gly His Leu Ile Ser Thr Ile Glu Met Ala Glu Leu Leu Thr Asp
            20                  25                  30

aga gat gaa cgc ctc tcg atc aca gtc atc atc atg aag ttg cca atg      144
Arg Asp Glu Arg Leu Ser Ile Thr Val Ile Ile Met Lys Leu Pro Met
        35                  40                  45

gag tca aaa acc gat ttc tat tcc cga aaa tcc aat tcg cgt ata cgt 192
Glu Ser Lys Thr Asp Phe Tyr Ser Arg Lys Ser Asn Ser Arg Ile Arg
    50                  55                  60

ttc ata gaa ttt tct ctc aac cag ccc att aca ccc aac aac ttt gtc 240
Phe Ile Glu Phe Ser Leu Asn Gln Pro Ile Thr Pro Asn Asn Phe Val
65                  70                  75                  80

act cat ttc atc gaa agc cac aag gat cct ata aga gat gct gtc acg 288
Thr His Phe Ile Glu Ser His Lys Asp Pro Ile Arg Asp Ala Val Thr
                85                  90                  95

aaa ata gtt cgt gat gag tcc aac tcg atc aga ctt gct gga ttc gtt 336
Lys Ile Val Arg Asp Glu Ser Asn Ser Ile Arg Leu Ala Gly Phe Val
            100                 105                 110

att gat atg ttt tgt act act atg att gac gta gcc aat gag ttt ggt 384
Ile Asp Met Phe Cys Thr Thr Met Ile Asp Val Ala Asn Glu Phe Gly
        115                 120                 125

gtc ccg act tat gtt ttc ttt aca act act gct gca ctg ctt ggg ttc 432
Val Pro Thr Tyr Val Phe Phe Thr Thr Thr Ala Ala Leu Leu Gly Phe
    130                 135                 140

acc ttc tat ttg cag agt cgc agc gat gaa cag aag ttg gat gtg aca 480
Thr Phe Tyr Leu Gln Ser Arg Ser Asp Glu Gln Lys Leu Asp Val Thr
145                 150                 155                 160

gaa tac aag aac tct aat gct gag tta ttg att ccc acg tac ata aat 528
Glu Tyr Lys Asn Ser Asn Ala Glu Leu Leu Ile Pro Thr Tyr Ile Asn
                165                 170                 175

cca gtt cct gct aac gta ttt cct tcg aga ttc ttt gat aag gat ggt 576
Pro Val Pro Ala Asn Val Phe Pro Ser Arg Phe Phe Asp Lys Asp Gly
            180                 185                 190

ttg gct atg ttc ctc ggt atg gca aga agg ttt aga gag acc aag gga 624
Leu Ala Met Phe Leu Gly Met Ala Arg Arg Phe Arg Glu Thr Lys Gly
        195                 200                 205

att atg att aac acc ttc ttg gat tta gaa gct cat gca atg aag tcc 672
Ile Met Ile Asn Thr Phe Leu Asp Leu Glu Ala His Ala Met Lys Ser
    210                 215                 220

ctc tca gat gat cat aca atc cca ccg gtc tac tca ata ggc cca ata 720
Leu Ser Asp Asp His Thr Ile Pro Pro Val Tyr Ser Ile Gly Pro Ile
225                 230                 235                 240

att cat gtt acg gct gaa aat gat gac gac aac aaa gat tat gac gag 768
Ile His Val Thr Ala Glu Asn Asp Asp Asp Asn Lys Asp Tyr Asp Glu
                245                 250                 255

atc atc aag tgg ctt cac gaa caa cct gtt tct tct gtt gtt ttt ctt 816
Ile Ile Lys Trp Leu His Glu Gln Pro Val Ser Ser Val Val Phe Leu
            260                 265                 270

tgc ttt gga agt atg gga ttt ttt gat gat gaa cag gtt aaa gag att 864
Cys Phe Gly Ser Met Gly Phe Phe Asp Asp Glu Gln Val Lys Glu Ile
        275                 280                 285

gcg gtt gca ctt gaa aag agt ggc cac cgg ttc ttg tgg tca ttg aga 912
Ala Val Ala Leu Glu Lys Ser Gly His Arg Phe Leu Trp Ser Leu Arg
    290                 295                 300

aag cct cct ccc aaa gac agg ttc gag tat cca tca gac tac gaa aat      960
Lys Pro Pro Pro Lys Asp Arg Phe Glu Tyr Pro Ser Asp Tyr Glu Asn
305                 310                 315                 320

ctt gaa gaa att ttg cca gaa ggg ttc ttg caa cgt act gca gga atc      1008
Leu Glu Glu Ile Leu Pro Glu Gly Phe Leu Gln Arg Thr Ala Gly Ile
                325                 330                 335

ggg aag gtg att gga tgg gct cca caa gtg gcc gtc tta tct cac cac      1056
Gly Lys Val Ile Gly Trp Ala Pro Gln Val Ala Val Leu Ser His His
            340                 345                 350

tcg gtg gga ggc ttt gtg tcg cat tgt ggt tgg aat tcg aca ctg gaa      1104
Ser Val Gly Gly Phe Val Ser His Cys Gly Trp Asn Ser Thr Leu Glu
        355                 360                 365

agt gtc tgg tgt gga gtg cca ata gca gca tgg cca atg tat gct gag      1152
Ser Val Trp Cys Gly Val Pro Ile Ala Ala Trp Pro Met Tyr Ala Glu
    370                 375                 380

cag cag act aat gcc ttc gag ttg gtg aag gac ttg gga att gct gtg      1200
Gln Gln Thr Asn Ala Phe Glu Leu Val Lys Asp Leu Gly Ile Ala Val
385                 390                 395                 400

gag att aag atg gac tat agg agg ggc agt gat gtg att gtg aag gct      1248
Glu Ile Lys Met Asp Tyr Arg Arg Gly Ser Asp Val Ile Val Lys Ala
                405                 410                 415

gaa gaa att gag aag gga atc agg cac cta atg gag cct gat agt gaa      1296
Glu Glu Ile Glu Lys Gly Ile Arg His Leu Met Glu Pro Asp Ser Glu
            420                 425                 430

atg agg aat aag atg aaa cag atg aaa aac aag agt aga ttg gct ttg      1344
Met Arg Asn Lys Met Lys Gln Met Lys Asn Lys Ser Arg Leu Ala Leu
        435                 440                 445

atg gaa ggt ggc tct tcc tac gat ttc ttg aga cat ttc atc gat aac      1392
Met Glu Gly Gly Ser Ser Tyr Asp Phe Leu Arg His Phe Ile Asp Asn
    450                 455                 460

att cca atg acg gat                                                  1407
Ile Pro Met Thr Asp
465


<210>  4
<211>  469
<212>  PRT
<213>  Forsythia koreana

<400>  4

Met Ala Glu Thr Lys Lys Ser Glu Leu Val Phe Ile Pro Val Pro Gly
1               5                   10                  15


Val Gly His Leu Ile Ser Thr Ile Glu Met Ala Glu Leu Leu Thr Asp
            20                  25                  30


Arg Asp Glu Arg Leu Ser Ile Thr Val Ile Ile Met Lys Leu Pro Met
        35                  40                  45


Glu Ser Lys Thr Asp Phe Tyr Ser Arg Lys Ser Asn Ser Arg Ile Arg

50                  55                  60

Phe Ile Glu Phe Ser Leu Asn Gln Pro Ile Thr Pro Asn Asn Phe Val
65                  70                  75                  80

Thr His Phe Ile Glu Ser His Lys Asp Pro Ile Arg Asp Ala Val Thr
                85                  90                  95

Lys Ile Val Arg Asp Glu Ser Asn Ser Ile Arg Leu Ala Gly Phe Val
            100                 105                 110

Ile Asp Met Phe Cys Thr Thr Met Ile Asp Val Ala Asn Glu Phe Gly
        115                 120                 125

Val Pro Thr Tyr Val Phe Phe Thr Thr Thr Ala Ala Leu Leu Gly Phe
    130                 135                 140

Thr Phe Tyr Leu Gln Ser Arg Ser Asp Glu Gln Lys Leu Asp Val Thr
145                 150                 155                 160

Glu Tyr Lys Asn Ser Asn Ala Glu Leu Leu Ile Pro Thr Tyr Ile Asn
                165                 170                 175

Pro Val Pro Ala Asn Val Phe Pro Ser Arg Phe Phe Asp Lys Asp Gly
            180                 185                 190

Leu Ala Met Phe Leu Gly Met Ala Arg Arg Phe Arg Glu Thr Lys Gly
        195                 200                 205

Ile Met Ile Asn Thr Phe Leu Asp Leu Glu Ala His Ala Met Lys Ser
    210                 215                 220

Leu Ser Asp Asp His Thr Ile Pro Pro Val Tyr Ser Ile Gly Pro Ile
225                 230                 235                 240

Ile His Val Thr Ala Glu Asn Asp Asp Asp Asn Lys Asp Tyr Asp Glu
                245                 250                 255

Ile Ile Lys Trp Leu His Glu Gln Pro Val Ser Ser Val Val Phe Leu
            260                 265                 270

Cys Phe Gly Ser Met Gly Phe Phe Asp Asp Glu Gln Val Lys Glu Ile
        275                 280                 285

Ala Val Ala Leu Glu Lys Ser Gly His Arg Phe Leu Trp Ser Leu Arg
    290                 295                 300

Lys Pro Pro Pro Lys Asp Arg Phe Glu Tyr Pro Ser Asp Tyr Glu Asn
305                 310                 315                 320

Leu Glu Glu Ile Leu Pro Glu Gly Phe Leu Gln Arg Thr Ala Gly Ile
                325                 330                 335

Gly Lys Val Ile Gly Trp Ala Pro Gln Val Ala Val Leu Ser His His
            340                 345                 350

Ser Val Gly Gly Phe Val Ser His Cys Gly Trp Asn Ser Thr Leu Glu
        355                 360                 365

Ser Val Trp Cys Gly Val Pro Ile Ala Ala Trp Pro Met Tyr Ala Glu
    370                 375                 380

Gln Gln Thr Asn Ala Phe Glu Leu Val Lys Asp Leu Gly Ile Ala Val
385                 390                 395                 400

Glu Ile Lys Met Asp Tyr Arg Arg Gly Ser Asp Val Ile Val Lys Ala
                405                 410                 415

Glu Glu Ile Glu Lys Gly Ile Arg His Leu Met Glu Pro Asp Ser Glu
            420                 425                 430

Met Arg Asn Lys Met Lys Gln Met Lys Asn Lys Ser Arg Leu Ala Leu
        435                 440                 445

Met Glu Gly Gly Ser Ser Tyr Asp Phe Leu Arg His Phe Ile Asp Asn
    450                 455                 460

Ile Pro Met Thr Asp
465

<210>  5
<211>  1404
<212>  DNA
<213>  Forsythia koreana

<220>
<221>  CDS
<222>  (1)..(1404)

<400>  5
atg gca gaa aca aag aaa tca gag ctt gtt ttc att cct gca cca gga      48
Met Ala Glu Thr Lys Lys Ser Glu Leu Val Phe Ile Pro Ala Pro Gly
1               5                   10                  15

ata ggt cac ttg ata tca act att gag ttg gct aag ctt ctc act gat      96
Ile Gly His Leu Ile Ser Thr Ile Glu Leu Ala Lys Leu Leu Thr Asp
            20                  25                  30

aga gat gag cac ctc tcg atc aca gtc ctc atc ttg aag ttg cca atg     144
Arg Asp Glu His Leu Ser Ile Thr Val Leu Ile Leu Lys Leu Pro Met
        35                  40                  45

gag tcg aaa acc gat tcc tat tcc caa aaa tcc aat tcg cgt ata cgt 192
Glu Ser Lys Thr Asp Ser Tyr Ser Gln Lys Ser Asn Ser Arg Ile Arg
    50                  55                  60

ttc ata gaa tta tct ctc aat caa cct att aca ccc aac aac ttt gtc 240
Phe Ile Glu Leu Ser Leu Asn Gln Pro Ile Thr Pro Asn Asn Phe Val
65                  70                  75                  80

act gat ttc atc gaa ggc cac aag gat cct ata aga gat gct gtc acg 288
Thr Asp Phe Ile Glu Gly His Lys Asp Pro Ile Arg Asp Ala Val Thr
                85                  90                  95

aaa ata gtt cgt gat gag tcc aac tct att aga ctt gct gga ttc gtt 336
Lys Ile Val Arg Asp Glu Ser Asn Ser Ile Arg Leu Ala Gly Phe Val
            100                 105                 110

att gat atg ttt tgt act act atg att gat gta gcc aat gag ttt ggt 384
Ile Asp Met Phe Cys Thr Thr Met Ile Asp Val Ala Asn Glu Phe Gly
        115                 120                 125

gtc ccg acg tat gtt ttc ttt aca act act gct gca atg ctt ggg ttc 432
Val Pro Thr Tyr Val Phe Phe Thr Thr Thr Ala Ala Met Leu Gly Phe
    130                 135                 140

atc ttc tat ttg cag agt cgc ggc gat gaa cag aag ttg gat gtg act 480
Ile Phe Tyr Leu Gln Ser Arg Gly Asp Glu Gln Lys Leu Asp Val Thr
145                 150                 155                 160

gag tac aag aac tca aat act aag tta ttg att ccc acg tac ata aat 528
Glu Tyr Lys Asn Ser Asn Thr Lys Leu Leu Ile Pro Thr Tyr Ile Asn
                165                 170                 175

ccg gtt cct gct aat gta ttt cct tct aaa tta ttt gat aag gat agt 576
Pro Val Pro Ala Asn Val Phe Pro Ser Lys Leu Phe Asp Lys Asp Ser
            180                 185                 190

ttg gct cca ttc gtc agt atg gca aga agg ttt aga gag acc aag gga 624
Leu Ala Pro Phe Val Ser Met Ala Arg Arg Phe Arg Glu Thr Lys Gly
        195                 200                 205

att ttg att aac acc ttc ttg gat tta gaa gct tat gca ttg aag tcc 672
Ile Leu Ile Asn Thr Phe Leu Asp Leu Glu Ala Tyr Ala Leu Lys Ser
    210                 215                 220

ctc tct gat gat cat aca atc cca ccg gtc tac tca ata ggc ccg ata 720
Leu Ser Asp Asp His Thr Ile Pro Pro Val Tyr Ser Ile Gly Pro Ile
225                 230                 235                 240

ctt cat gtt aag gtt gaa aat gat gac aaa aag aaa gat tat gac gag 768
Leu His Val Lys Val Glu Asn Asp Asp Lys Lys Lys Asp Tyr Asp Glu
                245                 250                 255

atc atc aat tgg ctt cac gaa caa cct gtt tcg tct gtt gtt ttt ctt 816
Ile Ile Asn Trp Leu His Glu Gln Pro Val Ser Ser Val Val Phe Leu
            260                 265                 270

tgc ttt gga agt ttg ggt tgt ttt gat gtc gaa cag gtt aaa gag att 864
Cys Phe Gly Ser Leu Gly Cys Phe Asp Val Glu Gln Val Lys Glu Ile
        275                 280                 285

gcg gtt gca ctt gaa aag agt ggc cac cgg ttc ttg tgg tca ttg aga 912
Ala Val Ala Leu Glu Lys Ser Gly His Arg Phe Leu Trp Ser Leu Arg
    290                 295                 300

aag cct cct ccc aaa gac ttc gag cat cca tca gac tac gaa aat ttt 960

Lys Pro Pro Pro Lys Asp Phe Glu His Pro Ser Asp Tyr Glu Asn Phe
305                 310                 315                 320

gaa gaa gta ttg cca gaa ggg ttc ttg caa cgt aca gca gga atc ggg      1008
Glu Glu Val Leu Pro Glu Gly Phe Leu Gln Arg Thr Ala Gly Ile Gly
                325                 330                 335

aag gtg att gga tgg gcg cca caa gtg gcc gtc tta tct cac cat tcg      1056
Lys Val Ile Gly Trp Ala Pro Gln Val Ala Val Leu Ser His His Ser
            340                 345                 350

gtg gga ggc ttt gtg tcg cat tgt ggt tgg aat tcg acg ctg gaa agt      1104
Val Gly Gly Phe Val Ser His Cys Gly Trp Asn Ser Thr Leu Glu Ser
        355                 360                 365

gtc tgg tgt gga gtg cca ata gcg gca tgg cca atg tat gcc gaa cag      1152
Val Trp Cys Gly Val Pro Ile Ala Ala Trp Pro Met Tyr Ala Glu Gln
    370                 375                 380

cag act aat gcc ttt gag ttg gtg aag gac ttg gga att gct gtg gag      1200
Gln Thr Asn Ala Phe Glu Leu Val Lys Asp Leu Gly Ile Ala Val Glu
385                 390                 395                 400

att aag atg gac tat agg aag ggc agt gat gtg att gtg aag gct gaa      1248
Ile Lys Met Asp Tyr Arg Lys Gly Ser Asp Val Ile Val Lys Ala Glu
                405                 410                 415

gaa att gag aag gga atc aag cac cta atg gag cct gat agt gaa atg      1296
Glu Ile Glu Lys Gly Ile Lys His Leu Met Glu Pro Asp Ser Glu Met
            420                 425                 430

agg aat aag atg aaa cag atg aaa agc aag agt aga ttg gct ttg atg      1344
Arg Asn Lys Met Lys Gln Met Lys Ser Lys Ser Arg Leu Ala Leu Met
        435                 440                 445

gaa ggt ggc tct tct tac aat ttc ttg agg cgt ttc atc gat aac att      1392
Glu Gly Gly Ser Ser Tyr Asn Phe Leu Arg Arg Phe Ile Asp Asn Ile
    450                 455                 460

cca atg acg gat                                                      1404
Pro Met Thr Asp
465

<210>  6
<211>  468
<212>  PRT
<213>  Forsythia koreana

<400>  6

Met Ala Glu Thr Lys Lys Ser Glu Leu Val Phe Ile Pro Ala Pro Gly
1               5                   10                  15

Ile Gly His Leu Ile Ser Thr Ile Glu Leu Ala Lys Leu Leu Thr Asp
            20                  25                  30

Arg Asp Glu His Leu Ser Ile Thr Val Leu Ile Leu Lys Leu Pro Met
        35                  40                  45

Glu Ser Lys Thr Asp Ser Tyr Ser Gln Lys Ser Asn Ser Arg Ile Arg
    50                  55                  60

Phe Ile Glu Leu Ser Leu Asn Gln Pro Ile Thr Pro Asn Asn Phe Val
65                  70                  75                  80

Thr Asp Phe Ile Glu Gly His Lys Asp Pro Ile Arg Asp Ala Val Thr
                85                  90                  95

Lys Ile Val Arg Asp Glu Ser Asn Ser Ile Arg Leu Ala Gly Phe Val
            100                 105                 110

Ile Asp Met Phe Cys Thr Thr Met Ile Asp Val Ala Asn Glu Phe Gly
        115                 120                 125

Val Pro Thr Tyr Val Phe Phe Thr Thr Thr Ala Ala Met Leu Gly Phe
    130                 135                 140

Ile Phe Tyr Leu Gln Ser Arg Gly Asp Glu Gln Lys Leu Asp Val Thr
145                 150                 155                 160

Glu Tyr Lys Asn Ser Asn Thr Lys Leu Leu Ile Pro Thr Tyr Ile Asn
                165                 170                 175

Pro Val Pro Ala Asn Val Phe Pro Ser Lys Leu Phe Asp Lys Asp Ser
            180                 185                 190

Leu Ala Pro Phe Val Ser Met Ala Arg Arg Phe Arg Glu Thr Lys Gly
        195                 200                 205

Ile Leu Ile Asn Thr Phe Leu Asp Leu Glu Ala Tyr Ala Leu Lys Ser
    210                 215                 220

Leu Ser Asp Asp His Thr Ile Pro Pro Val Tyr Ser Ile Gly Pro Ile
225                 230                 235                 240

Leu His Val Lys Val Glu Asn Asp Asp Lys Lys Lys Asp Tyr Asp Glu
                245                 250                 255

Ile Ile Asn Trp Leu His Glu Gln Pro Val Ser Ser Val Val Phe Leu
            260                 265                 270

Cys Phe Gly Ser Leu Gly Cys Phe Asp Val Glu Gln Val Lys Glu Ile
        275                 280                 285

Ala Val Ala Leu Glu Lys Ser Gly His Arg Phe Leu Trp Ser Leu Arg
    290                 295                 300

Lys Pro Pro Pro Lys Asp Phe Glu His Pro Ser Asp Tyr Glu Asn Phe
305                 310                 315                 320

Glu Glu Val Leu Pro Glu Gly Phe Leu Gln Arg Thr Ala Gly Ile Gly
                325                 330                 335

Lys Val Ile Gly Trp Ala Pro Gln Val Ala Val Leu Ser His His Ser
            340                 345                 350

Val Gly Gly Phe Val Ser His Cys Gly Trp Asn Ser Thr Leu Glu Ser
        355                 360                 365

Val Trp Cys Gly Val Pro Ile Ala Ala Trp Pro Met Tyr Ala Glu Gln
    370                 375                 380

Gln Thr Asn Ala Phe Glu Leu Val Lys Asp Leu Gly Ile Ala Val Glu
385                 390                 395                 400

Ile Lys Met Asp Tyr Arg Lys Gly Ser Asp Val Ile Val Lys Ala Glu
                405                 410                 415

Glu Ile Glu Lys Gly Ile Lys His Leu Met Glu Pro Asp Ser Glu Met
            420                 425                 430

Arg Asn Lys Met Lys Gln Met Lys Ser Lys Ser Arg Leu Ala Leu Met
        435                 440                 445

Glu Gly Gly Ser Ser Tyr Asn Phe Leu Arg Arg Phe Ile Asp Asn Ile
    450                 455                 460

Pro Met Thr Asp
465

<210>  7
<211>  30
<212>  DNA
<213>  Artificial

<220>
<223>  Synthetic DNA

<400>  7
cacccatatg gcagaaacaa agaaatcaga                                        30

<210>  8
<211>  29
<212>  DNA
<213>  Artificial

<220>
<223>  Synthetic DNA

<400>  8
agatctttaa tccgtcattg gaatgttat                                         29

<210>  9
<211>  21
<212>  DNA
<213>  Artificial

<220>
<223>  Synthetic DNA

<400>  9
tagcggatca accaactaaa c                                                21


<210>  10
<211>  21
<212>  DNA
<213>  Artificial

<220>
<223>  Synthetic DNA

<400>  10
tcttgccata ccgaggaaca t                                                21


<210>  11
<211>  21
<212>  DNA
<213>  Artificial

<220>
<223>  Synthetic DNA

<400>  11
tagcagatca acccagtaaa t                                                21


<210>  12
<211>  21
<212>  DNA
<213>  Artificial

<220>
<223>  Synthetic DNA

<400>  12
tcttgccata ctgacgaatg g                                                21


<210>  13
<211>  22
<212>  DNA
<213>  Artificial

<220>
<223>  Synthetic DNA

<400>  13
taaaacgact ctcggcaacg ga                                               22


<210>  14
<211>  23
<212>  DNA
<213>  Artificial

<220>
<223>  Synthetic DNA

<400>  14
atcccgcctg acctgggtc gct                                                23

## Description

### FIELD OF THE INVENTION

The present invention relates to a Forsythia koreana-derived glucosyltransferase specific to the position-4 of a furofuran-type lignan (furofuran lignan 4-O-glucosyltransferase), a polynucleotide encoding the enzyme, a vector comprising the polynucleotide, a transformant obtained using the vector; and so on.

### BACKGROUND ART

Lignans are a class of plant secondary metabolites ubiquitously distributed in the plant kingdom (Non-Patent Literature 1: Umezawa, T. (2003) Phytochem. Rev. 2, 371-390). Some lignans have useful biological activities and are used as drugs or health foods. For example, main furofuran-type lignans contained in Sesamum indicum of the family Pedaliaceae are known to have functions including antioxidative properties, improvement of lipid metabolism, protection of liver function, etc. and are commercially available (Non-Patent Literature 2: Nakai, M. et al. (2003) J. Agri. Food Chem. 51, 1666-1670; Non-Patent Literature 3: Tsuruoka, T. et al. (2005) Biosci. Biotech. Biochem. 69, 179-188; Non-Patent Literature 4: Sirato-Yasumoto, S. et al. (2001) J. Agri. Food Chem. 49, 2647-2651). On the other hand, podophyllotoxins, which are allyltetralin lactone type anti-tumor lignans contained in the root of Berberidaceae Podophyllum peltatum (American May apple), have a cell division inhibitory activity and are used as carcinostatic agents and for development of novel carcinostatic agents (Non-Patent Literature 5: Ayres, D. C. and Loike, J. D. (1990) Lignans: Chemical Biological and Clinical Properties. Cambridge Univ. Press, Cambridge, U.K.). Also, numerous lignans are considered to be metabolized in vivo, after their uptake, into enterolactone which has a female sex hormone-like activity. Lignans are secondary metabolites to which attention has been drawn in recent years (Non-Patent Literature 6: Heinonen, S. et al. (2001) J. Agric. Food Chem. 49, 3178-3186).

Forsythia koreana (genus Forsythia) of the family Oleaceae, which is a perennial woody plant, is strong and easy to cultivate, bears yellow bright flowers and is often used as a garden shrub. On the other hand, it is known that lignans having an antioxidative or anti-inflammatory activity are abundantly accumulated in the leaves, seeds and petals of Forsythia koreana, and in Asia (especially in China and Japan) the leaves are drunk as tea (Non-Patent Literature 7: Guo, H. et al. (2007) Rapid Communications in Mass Spectrometry 21, 715-729; Non-Patent Literature 8: Chang, M-J. et al. (2008) Biosci. Biotech. Biochem. 72, 2750-2755). Unlike annual herbaceous plants such as sesame or linseed, there is no need to seed or raise seedlings of Forsythia koreana every year. It is possible to obtain functional lignans from the leaves over several years. Forsythia koreana is thus considered to be a target for molecular breeding in beneficial secondary metabolites.

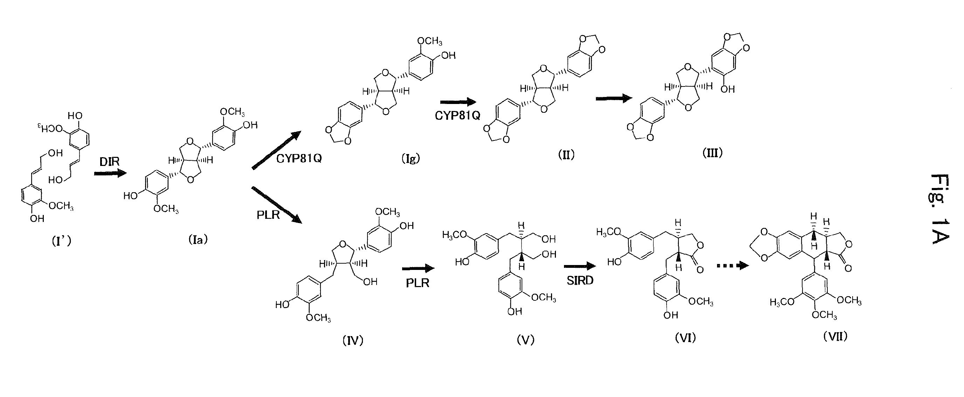

Lignan biosynthesis in Forsythia koreana of the family Oleaceae starts by formation of the furofuran-type lignan of structural formula (Ia) (cf. FIG. 1A; hereinafter also the same as for the other structural formulae) via oxidative polymerization of the monolignol of structural formula (I') (Non-Patent Literature 9: Davin, L. B. and Lewis, N. G. (2003) Phytochem. Rev. 2, 257-288; Non-Patent Literature 10: Suzuki, S. and Umezawa, T. (2007) J. Wood. Sci. 53, 273-284). The lignan of structural formula (1a) is then converted, via the lignans of structural formulae (IV) and (V) (Non-Patent Literature 11: Dinkova-Kostova, A. et al. (1996) J. Biol. Chem. 271, 29473-29482; Non-Patent Literature 12: Nakatsubo, T. et al. (2008) J. Biol. Chem. 283, 15550-1557; Non-Patent Literature 13: Min, T. et al. (2003) J. Biol. Chem. 278, 50714-50723; Non-Patent Literature 14: Gang, D. et al. (1999) J. Biol. Chem. 274, 7516-7527), into the lignan of structural formula (VI) (Non-Patent Literature 15: Xia, Z-Q. et al (2001) J. Biol. Chem. 276, 12614-12623; Non-Patent Literature 16: Youn, B. et al (2005) J. Biol. Chem. 280, 12917-12926). After this step until the lignan glycoside of structural formula (VII), it is necessary to further perform multiple enzyme reactions. In a series of such lignan biosynthetic systems in Forsythia koreana, only some enzyme activities are confirmed but catalytic enzymes responsible for the respective reactions or genes encoding the same have not been isolated yet. Although all lignans contained in Forsythia koreana are stably present as the glycosides, only the activity of transferring sugar to the position 7 of the lignan of structural formula (VII) is detected (Non-Patent Literature 17: Berim, A. et al. (2008) Phytochemistry 69, 374-381) but other lignan glycosyltransferases are not identified. On the other hand, most of the lignans in Sesamum indicum are present as glycosides but only glycosyltransferase UGT71A9 to the lignan shown by structural formula (III) is identified. This enzyme is inactive with the positions other than the position 2 or with the other lignan compounds. The other lignan glycosyltransferases still remain unclear (Non-Patent Literature 18: Ono, E. et al. (2006) Proc. Natl. Acad. Sci. USA 103, 10116-10121; Non-Patent Literature 19: Noguchi, A., et al. (2008) Plant J. 54, 415-427).

In general, the regioselectivity of glycosyltransferases in plants is high. The amino acid sequence between glycosyltransferases that react with the same or similar compound at the same position is conserved across the species. It is therefore possible to isolate the enzyme using sequence homology. Even if compounds are the same or similar, however, a glycosyltransferase that adds sugar to a different position has low homology in its amino acid sequence, and it is extremely difficult to isolate the same based on the sequence information of glycosyltransferase which reacts at other positions. Accordingly, it was extremely difficult to assume the structure of a glycosyltransferase that adds sugar to the other lignan compound, for example, to the position 4 and/or position 4' of the furofuran-type lignan of structural formula (Ia), etc., based on the Sesamum indicum-derived UGT71A9 that adds sugar to the position 2 of the compound of structural formula (III) described above, and isolate the same.

### PRIOR ART LITERATURES

### Non-Patent Literatures

[Non-Patent Literature 1] Umezawa, T. (2003) Phytochem. Rev. 2, 371-390
[Non-Patent Literature 2] Nakai, M. et al. (2003) J. Agri. Food Chem. 51, 1666-1670
[Non-Patent Literature 3] Tsuruoka, T. et al. (2005) Biosci. Biotech. Biochem. 69, 179-188
[Non-Patent Literature 4] Sirato-Yasumoto, S. et al. (2001) J. Agri. Food Chem. 49, 2647-2651
[Non-Patent Literature 5] Ayres, D. C. and Loike, J. D. (1990) Lignans: Chemical Biological and Clinical Properties. Cambridge Univ. Press, Cambridge, U.K.
[Non-Patent Literature 6] Heinonen, S. et al. (2001) J. Agric. Food Chem. 49, 3178-3186
[Non-Patent Literature 7] Guo, H. et al. (2007) Rapid Communications in Mass Spectrometry 21, 715-729
[Non-Patent Literature 8] Chang, M-J. et al. (2008) Biosci. Biotech. Biochem. 72, 2750-2755
[Non-Patent Literature 9] Davin, L. B. and Lewis, N. G. (2003) Phytochem. Rev. 2, 257-288
[Non-Patent Literature 10] Suzuki, S. and Umezawa, T. (2007) J. Wood. Sci. 53, 273-284
[Non-Patent Literature 11] Dinkova-Kostova, A. et a1. (1996) J. Biol. Chem. 271, 29473-29482
[Non-Patent Literature 12] Nakatsubo, T. et al. (2008) J. Biol. Chem. 283, 15550-1557
[Non-Patent Literature 13] Min, T. et al. (2003) J. Biol. Chem. 278, 50714-50723
[Non-Patent Literature 14] Gang, D. et al. (1999) J. Biol. Chem. 274, 7516-7527
[Non-Patent Literature 15] Xia, Z-Q. et al (2001) J. Biol. Chem. 276, 12614-12623
[Non-Patent Literature 16] Youn, B. et al (2005) J. Biol. Chem. 280, 12917-12926
[Non-Patent Literature 17] Berim, A. et al. (2008) Phytochemistry 69, 374-381
[Non-Patent Literature 18] Ono, E. et al. (2006) Proc. Natl. Acad. Sci. USA 103, 10116-10121
[Non-Patent Literature 19] Noguchi, A., et al. (2008) Plant J. 54, 415-427

### SUMMARY OF THE INVENTION

Under these circumstances, it has been desired to isolate and identify a glycosyltransferase, which adds sugar to the position 4 and/or 4' offurofuran-type lignans.

Taking the foregoing circumstances into account, the present invention has been made and provides a polynucleotide (a polynucleotide encoding a furofuran lignan 4-0-glucosyltransferase), a protein (a furofuran lignan 4-O-glucosyltransferase), a vector comprising the polynucleotide, a transformant wherein the vector is introduced, and so on.
(1) A polynucleotide of any one of (a) to (f) below:
   (a) a polynucleotide comprising a polynucleotide consisting of the nucleotide sequence represented by SEQ ID NO: 3 or 5;
   (b) a polynucleotide comprising a polynucleotide encoding a protein consisting of the amino acid sequence represented by SEQ ID NO: 4 or 6;
   (c) a polynucleotide comprising a polynucleotide encoding a protein consisting of the amino acid sequence wherein 1 to 15 amino acids are deleted, substituted, inserted and/or added in the amino acid sequence represented by SEQ ID NO: 4 or 6, and having a glucosyltransferase activity against a lignan;
   (d) a polynucleotide comprising a polynucleotide encoding a protein that has an amino acid sequence having a homology of at least 80% to the amino acid sequence represented by SEQ ID NO: 4 or 6, and has a glucosyltransferase activity against a lignan;
   (e) a polynucleotide comprising a polynucleotide encoding a protein that hybridizes under stringent conditions with a polynucleotide consisting of a nucleotide sequence complementary to the nucleotide sequence represented by SEQ ID NO: 3 or 5, and has a glucosyltransferase activity against a lignan; or,
   (f) a polynucleotide comprising a polynucleotide encoding a protein that hybridizes under stringent conditions with a polynucleotide consisting of a nucleotide sequence complementary to the nucleotide sequence of a polynucleotide encoding a protein consisting of the amino acid sequence represented by SEQ ID NO: 4 or 6, and has a glucosyltransferase activity against a lignan.
(2) The polynucleotide of (1) described above, which is any one of (g) to (j) below:
   (g) a polynucleotide comprising a polynucleotide encoding a protein consisting of the amino acid sequence wherein not exceeding 10 amino acids are deleted, substituted, inserted and/or added in the amino acid sequence represented by SEQ ID NO: 4 or 6, and having a glucosyltransferase activity against a lignan;
   (h) a polynucleotide comprising a polynucleotide encoding a protein that has an amino acid sequence having a homology of at least 90% to the amino acid sequence represented by SEQ ID NO: 4 or 6, and has a glucosyltransferase activity against a lignan;
   (i) a polynucleotide comprising a polynucleotide encoding a protein that hybridizes under high stringent conditions with a polynucleotide consisting of a nucleotide sequence complementary to the nucleotide sequence represented by SEQ ID NO: 3 or 5, and has a glucosyltransferase activity against a lignan; or,
   (j) a polynucleotide comprising a polynucleotide encoding a protein that hybridizes under high stringent conditions with a polynucleotide consisting of a nucleotide sequence complementary to the nucleotide sequence of a polynucleotide encoding a protein consisting of the amino acid sequence represented by SEQ ID NO: 4 or 6, and has a glucosyltransferase activity against a lignan.

In the polynucleotides of (1) and (2) described above, the glucosyltransferase activity against lignans includes, for example, a glucosyltransferase activity against a furofuran-type lignan, more specifically, a glucosyltransferase activity against the hydroxy group at position 4 and/or position 4' of furofuran-type lignans. Herein, the furofuran-type lignan includes, for example, the lignan shown by structural formula (Ia), (Ib) or (Ic) below.

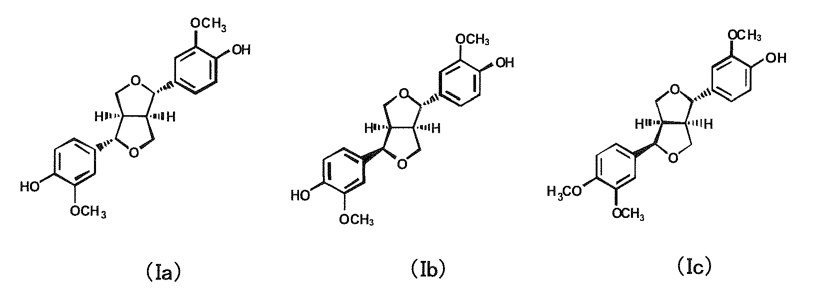

The polynucleotide of (1) described above includes, for example, a polynucleotide consisting of the nucleotide sequence represented by SEQ ID NO: 3 or 5 and a polynucleotide encoding a protein consisting of the amino acid sequence represented by SEQ ID NO: 4 or 6.

The polynucleotides of (1) and (2) described above include, for example, those which are DNA.
(3) A protein encoded by the polynucleotide of (1) or (2) above.
(4) A vector comprising the polynucleotide of (1) or (2) above.
(5) A transformant, wherein the polynucleotide of (1) or (2) above is introduced.
(6) A transformant, wherein the vector of (4) above is introduced.
(7) A method for producing the protein of (3) above, which comprises using the transformant of (5) or (6) above.
(8) A method for producing a glucoside, which comprises forming a glucoside from UDP-glucose and a glycosyl acceptor substrate using the protein of (3) above as a catalyst.

In the method of (8) described above, the glycosyl acceptor substrate includes, for example, a furofuran-type lignan. Herein, the furofuran-type lignan includes, for example, the lignan shown by structural formula (Ia), (Ib) or (Ic) described above.

### EFFECTS OF THE INVENTION

According to the present invention, the invention can provide a glycosyltransferase capable of transferring glucose to the position 4 and/or position 4' of furofuran-type lignans. According to the present invention, the invention can further provide a polynucleotide encoding the enzyme, a vector comprising the polynucleotide, a transformant obtained using the vector, a method for producing a glucoside using the enzyme, and so on.

According to the present invention, glucose can be transferred to the position 4 and/or position 4' offurofuran-type lignans by introducing the gene of the invention in vitro or into a host cell, and useful lignan glycosides which can contribute to the development of new functional food materials, molecular breeding of secondary metabolism, etc. can be easily produced. In this aspect, the present invention is extremely useful.

### BRIEF DESCRIPTION OF THE DRAWINGS

FIG. 1A shows biosynthetic pathways (metabolic pathways) of the lignans from Forsythia koreana and Sesamum indicum. "DIR" denotes a protein involved in the reaction that the lignan of structural formula (Ia) is formed from the monolignol of structural formula (I'), "PLR" denotes an enzyme which catalyzes the reaction that the lignans of structural formula (IV) and structural formula (V) are formed from the lignan of structural formula (Ia), "SIRD" denotes an enzyme which catalyzes the reaction that the lignan of structural formula (VI) is formed from the lignan of structural formula (V), and "CYP81Q" denotes an enzyme which catalyzes the reaction that the lignans of structural formula (Ig) and structural formula (II) are formed from the lignan of structural formula (Ia). For the monolignol of structural formula (I') and the lignans of structural formulae (Ia), (Ig) and (II) through (VII), reference can be made on the structural formulae in FIG. IA.

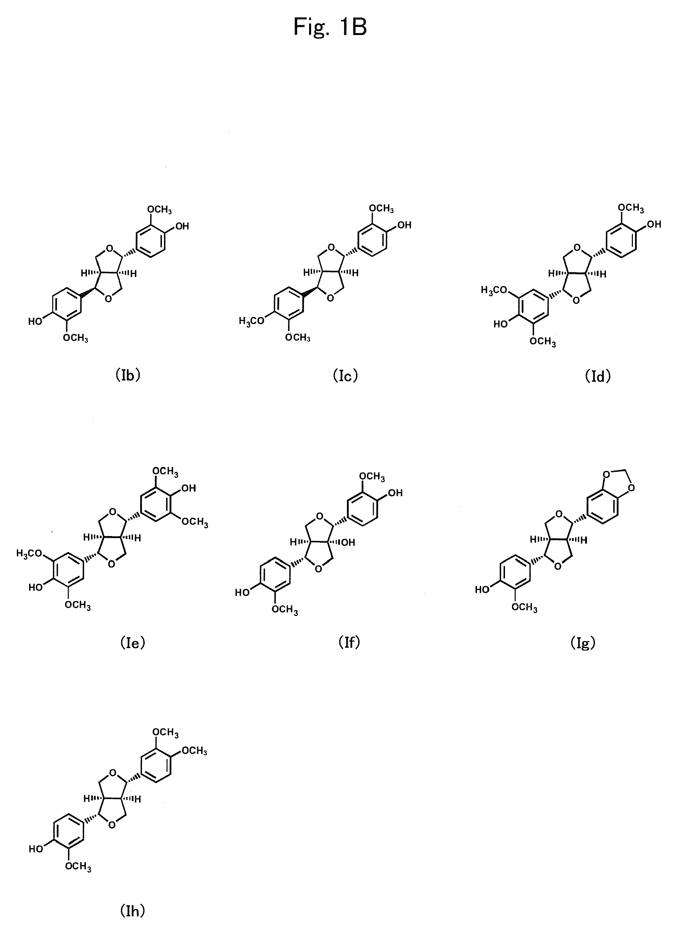

FIG. 1B shows the structures of various furofuran-type lignans which are similar to the lignan of structural formula (Ia). For the lignans of structural formulae (Ib), (Ic), (Id), (Ie), (If), (Ig) and (Ih), reference can be made on the structural formulae in FIG. IB (for the lignan of structural formula (Ig), reference may be made on the structural formulae in FIG. IA as stated above).

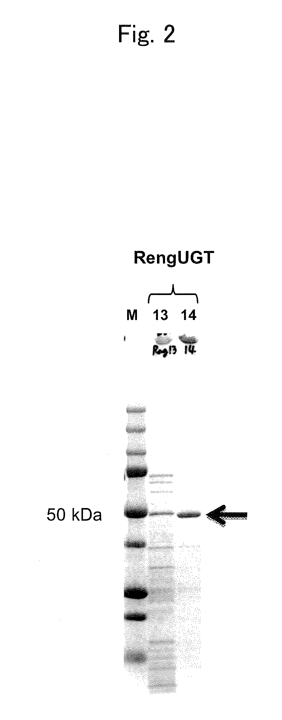

FIG. 2 shows the results of SDS-PAGE (200 mM imidazole eluate) in EXAMPLE 3. The arrow denotes furofuran lignan 4-0-glucosyltransferases (clone 13: RengUGT3, clone 14: RengUGT4), which are glucosyltransferase (RengUGT) proteins derived from the Escherichia coli expression Forsythia koreana detected at the size of about 50 KDa.

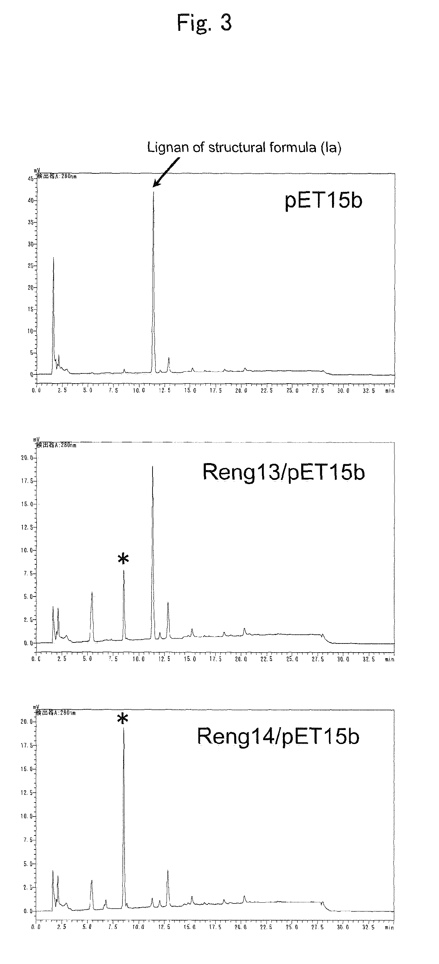

FIG. 3 shows the results of HPLC analysis in EXAMPLE 3. The HPLC analysis charts for the enzyme reaction solution at A280 nm are shown. In the order from above downward, the charts show the results of the reaction between an empty vector and the lignan of structural formula (Ia) (pET15b), the results of the reaction between RengUGT13 and the lignan of structural formula (Ia) (Reng13/pET15b), and the results of the reaction between RengUG14 and the lignan of structural formula (Ia) (Reng14/pET15b). The peaks with asterisk represent the peaks of the newly detected products.

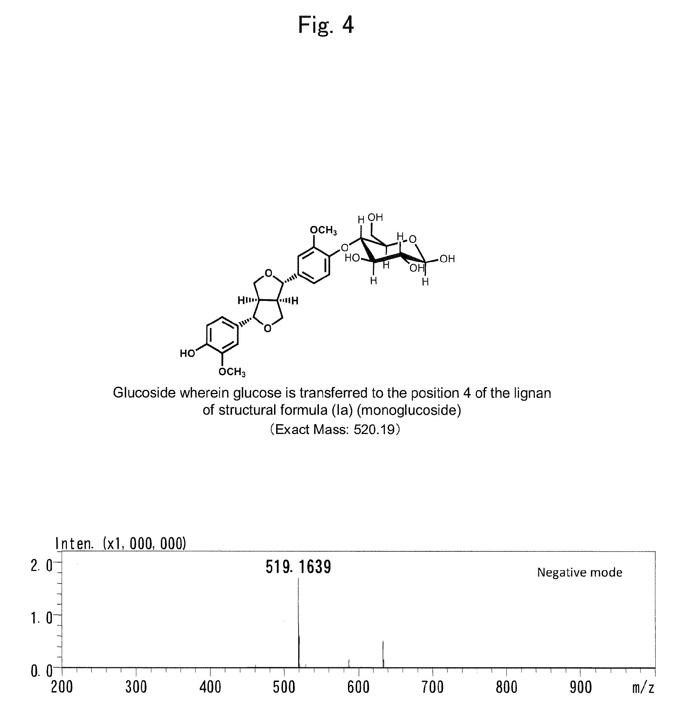

FIG. 4 shows the results of LC-MS analysis in EXAMPLE 3. The upper row denotes the structure of monoglucoside in which one molecule of glucose is transferred to the position 4 of the lignan of structural formula (Ia). The lower row denotes MS spectra of the enzyme product in a negative mode.

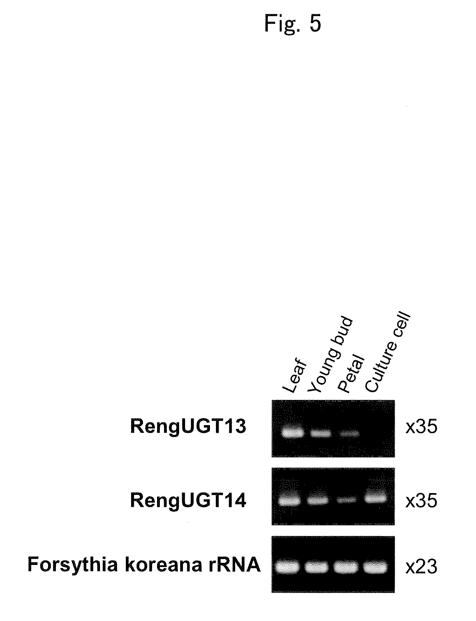

FIG. 5 shows the results of RT-PCR analysis in EXAMPLE 4. The figure shows the expression profile of the gene from Forsythia koreana in each organ. The results of gene expression of RengUGT13, RengUGT14 and Forsythia koreana rRNA are shown in the order from above downward. The numerical values on the right side (e.g., x35) designate the number of reaction cycles for PCR.

### BEST MODE FOR CARRYING OUT THE INVENTION

Hereinafter, the present invention is described in detail. The scope of the present invention is not restricted to these explanations, but appropriate modifications and implementation can also be made for other examples than those which are discussed below, without departing from the gist of the invention. The specification embraces, in its entirety, Japanese Patent Application No. 2009-120637 (filed May 19, 2009), based on which the priority of this application is claimed. All publications cited in the specification, for example, prior art literatures and published patent applications, patents and other patent literatures are hereby incorporated by reference in their entirety.

The present inventors screened glucosyltransferase (UGT; UDP-glucosyltransferase)-like genes from Forsythia koreana, obtained six Forsythia koreana UGT (RengUGT) candidate genes, and isolated and identified RengUGT13 and RengUGT14 from these Escherichia coli expression proteins as glycosyltransferases having a novel activity of transferring glucose specifically to the position 4 and/or position 4' of furofuran-type lignans represented by the lignans of structural formulae (Ia), (Ib) and (Ic).

### 1. Polynucleotide of the Invention

First, the present invention provides (a) a polynucleotide comprising a polynucleotide consisting of the nucleotide sequence of SEQ ID NO: 3 or 5 (specifically, a DNA; hereinafter sometimes briefly referred to as "DNA"); and (b) a polynucleotide comprising a polynucleotide encoding a protein having the amino acid sequence of SEQ ID NO: 4 or 6. The DNA intended in the present invention is not limited to the DNA encoding the furofuran lignan 4-0-glucosyltransferases (e.g., RengUGT3 and RengUGT14) described above, but includes other DNAs encoding proteins functionally equivalent to this protein.

The functionally equivalent protein is, for example, (c) a protein consisting of the amino acid sequence represented by SEQ ID NO: 4 or 6, in which 1 to 15 amino acids are deleted, substituted, inserted and/or added and having a glucosyltransferase activity against lignans. Such a protein includes, for example, a protein consisting of the amino acid sequence represented by SEQ ID NO: 4 or 6 wherein 1 to 15, 1 to 14, 1 to 13, 1 to 12, 1 to 11, 1 to 10, 1 to 9, 1 to 8, 1 to 7, 1 to 6 (1 to several), 1 to 5, 1 to 4, 1 to 3, 1 to 2 or 1 amino acid(s) is/are deleted, substituted, inserted and/or added and having the glucosyltransferase activity against lignans. In general, the smaller the number of deletions, substitutions, insertions, and/or additions of the amino acid residues is, the more preferable it is.

The functionally equivalent protein also includes, for example, (d) a protein having an amino acid sequence having at least 80% homology to the amino acid sequence represented by SEQ ID NO: 4 or 6 and having a glucosyltransferase activity against lignans. Such a protein includes a protein having an amino acid sequence having a homology of approximately 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher, to the amino acid sequence represented by SEQ ID NO: 4 or 6, and having the glucosyltransferase activity against lignans. In general, the higher the above homology percentage is, the more preferable it is.

As used herein, the term "glucosyltransferase activity against lignans" is used to mean an activity of catalyzing the reaction that involves the transfer of glucose to furofuran-type lignans as a glycosyl acceptor substrate, dependently on a glycosyl donor UDP-glucose to produce a glucoside. For example, the term means a furofuran lignan 4-0-glucosyltransierase (e.g., RengUGT13 and RengUGT14) activity, which catalyzes the reaction of transferring glucose to the hydroxy group at the position 4 and/or position 4' of furofuran-type lignans dependently on UDP-glucose to form a glucoside. As used herein, the enzymes shown by the term "furofuran lignan 4-O-glucosyltransferases" are capable of transferring glucose to the hydroxy group at the position 4 and/or position 4', as described above, but are not limited to the enzymes which transfer glucose only to the hydroxy group at the position 4. Herein, the position 4 and/or position 4' of furofuran-type lignans are intended to mean the position 4 and/or position 4' as defined by the IUPAC nomenclature.

The glucosyltransferase activity against lignans can be assayed, for example, by reacting UDP-glucose with the glycosyl acceptor substrate furofuran-type lignan (e.g., the lignans represented by structural formulae (Ia), (Ib) and (Ic), etc.) in the presence of an enzyme to be assayed and analyzing the resulting reaction product by HPLC, etc. (of., EXAMPLES later described for more details).

The present invention further includes (e) a polynucleotide comprising a polynucleotide encoding a protein that hybridizes under stringent conditions with a polynucleotide consisting of a nucleotide sequence complementary to the nucleotide sequence represented by SEQ ID NO: 3 or 5 and has the glucosyltransferase activity against lignans.

The present invention also includes (f) a polynucleotide comprising a polynucleotide encoding a protein that hybridizes under stringent conditions with a polynucleotide consisting of a nucleotide sequence complementary to the nucleotide sequence of a polynucleotide encoding a protein consisting of the amino acid sequence represented by SEQ ID NO: 4 or 6 and has the glucosyltransferase activity against lignans.

As used herein, the term "polynucleotide" is used to mean a DNA or RNA, preferably a DNA.

As used herein, the term "polynucleotide that hybridizes under stringent conditions" refers to, for example, a polynucleotide consisting of a nucleotide sequence complementary to the nucleotide sequence represented by SEQ ID NO: 3 or 5; or a polynucleotide obtained by colony hybridization, plaque hybridization, Southern hybridization or the like, using as a probe all or part of a polynucleotide consisting of a nucleotide sequence complementary to a nucleotide sequence of a polynucleotide encoding the amino acid sequence represented by SEQ ID NO: 4 or 6. The hybridization method which may be used includes methods described in, for example, "Sambrook & Russell, Molecular Cloning: A Laboratory Manual Vol. 3, Cold Spring Harbor, Laboratory Press 2001," "Ausubel, Current Protocols in Molecular Biology, John Wiley & Sons 1987-1997," etc.

As used herein, the term "stringent conditions" may be any of low stringent conditions, moderate stringent conditions and high stringent conditions. The "low stringent conditions" are, for example, the conditions of 5x SSC, 5x Denhardt's solution, 0.5% SDS, 50% formamide and 32°C. The "moderate stringent conditions" are, for example, the conditions of 5x SSC, 5x Denhardt's solution, 0.5% SDS, 50% formamide and 42°C. The "high stringent conditions" are, for example, the conditions of 5x SSC, 5x Denhardt's solution, 0.5% SDS, 50% formamide and 50°C. Under these conditions, as the temperature is higher, a DNA with higher homology is expected to be obtained efficiently at higher temperature, although multiple factors are involved in the hybridization stringency including temperature, probe concentration, probe length, ionic strength, time, salt concentration and the like. A person skilled in the art may achieve a similar stringency by appropriately choosing these factors.

In the case that a commercially available kit is used for the hybridization, for example, Alkphos Direct Labeling Reagents (manufactured by Amersham Pharmacia) can be used. In this case, according to the attached protocol, a membrane is incubated with a labeled probe overnight, the membrane is washed with a primary wash buffer containing 0.1 % (w/v) SDS at 55°C and the hybridized DNA can then be detected.

In addition to those described above, other polynucleotides that can be hybridized include DNAs having a homology of approximately 60% or higher, approximately 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher. 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher, to a DNA encoding the nucleotide sequence of SEQ ID NO: 3 or a DNA encoding the amino acid sequence represented by SEQ ID NO: 4, as calculated by homology search software, such as FASTA and BLAST using default parameters.

Homology between amino acid sequences or nucleotide sequences can be determined by using algorithm BLAST by Karlin and Altschul (Proc. Natl. Acad. Sci. USA, vol. 87, p. 2264-2268, 1990; Proc. Natl. Acad. Sci. USA, vol. 90, p. 5873, 1993). The programs called BLASTN and BLASTX based on BLAST algorithm have been developed (Altschul SF, et al., J. Mol. Biol., vol. 215, p. 403, 1990). When a nucleotide sequence is sequenced using BLASTN, the parameters are set at, for example, score = 100 and word length =12. When an amino acid sequence is sequenced using BLASTX, the parameters are set at, for example, score = 50 and word length = 3. When BLAST and Gapped BLAST programs are used, default parameters for each of the programs are employed.

The polynucleotide of the present invention described above can be acquired by known genetic engineering techniques or known synthetic techniques.

### 2. Protein of the Invention

In yet another embodiment, the present invention also provides the protein encoded by the polynucleotide of the present invention described above. The protein in an embodiment of the present invention is a protein consisting of the amino acid sequence represented by SEQ ID NO: 4 or 6. The protein in another embodiment of the present invention is a protein having the amino acid sequence represented by SEQ ID NO: 4 or 6.

The protein in yet another embodiment of the present invention is a protein consisting of the amino acid sequence represented by SEQ ID NO: 4 or 6, in which 1 to 15 amino acids are deleted, substituted, inserted and/or added and having the glucosyltransferase activity against lignans. Such a protein includes a protein having the amino acid sequence having the homology described above to the amino acid sequence represented by SEQ ID NO: 4 or 6 and having the glucosyltransferase activity against lignans. These proteins may be obtained by using site-directed mutagenesis described in "Sambrook & Russell, Molecular Cloning: A Laboratory Manual Vol. 3, Cold Spring Harbor, Laboratory Press 2001," "Ausubel, Current Protocols in Molecular Biology, John Wiley & Sons 1987-1997," "Nuc. Acids Res., vol. 10, p. 6487, 1982," "Proc. Natl. Acad. Sci. USA, vol. 79, p. 6409, 1982," "Gene, vol. 34, p. 315, 1985", "Nuc. Acids Res., vol. 13, p. 4431, 1985," "Proc. Natl. Acad. Sci. USA, vol. 82, p. 488, 1985," etc.

In the amino acid sequence for the protein of the present invention, the deletion, substitution, insertion and/or addition of one or more (e.g., 1 to 15, preferably 10 or less) amino acid residues is intended to mean that one or a plurality of amino acid residues are deleted, substituted, inserted and/or added at one or a plurality of positions in the same amino acid sequence. Two or more types of deletion, substitution, insertion and addition may occur concurrently.

Examples of amino acid residues which are mutually substitutable are given below. Amino acid residues in the same group are mutually substitutable. Group A: leucine, isoleucine, norleucine, valine, norvaline, alanine, 2-aminobutanoic acid, methionine, o-methylserine, t-butylglycine, t-butylalanine and cyclohexylalanine; Group B: aspartic acid, glutamic acid, isoaspartic acid, isoglutamic acid, 2-aminoadipic acid and 2-aminosuberic acid; Group C: asparagine and glutamine; Group D: lysine, arginine, ornithine, 2,4-diaminobutanoic acid and 2,3-diaminopropionic acid; Group E: proline, 3-hydroxyproline and 4-hydroxyproline; Group F: serine, threonine and homoserine; and Group G: phenylalanine and tyrosine.

The protein of the present invention may also be produced by chemical synthesis methods such as the Fmoc method (fluorenylmethyloxycarbonyl method), the tBoc method (t-butyloxycarbonyl method), or the like. In addition, peptide synthesizers available from Advanced ChemTech, Perkin Elmer, Pharmacia, Protein Technology Instrument, Synthecell-Vega, PerSeptive, Shimadzu Corp., etc. may also be used for the chemical synthesis.

Herein, the protein of the present invention is the furofuran lignan 4-O-glucosyltransferase. The "glucosyltransferase" catalyzes the reaction of transferring a glucose residue from a glycosyl donor to a glycosyl acceptor substrate to form a glucoside. In the present invention, the glycosyl acceptor substrate is, for example, a furofuran-type lignan, and the glycosyl donor is, e.g., UDP-glucose. In an embodiment of the present invention, the protein catalyzes the reaction of transferring a glucose residue from UDP-glucose to the position 4 and/or position 4' of a glycosyl acceptor substrate furofuran-type lignan (e.g., the lignans of structural formulae (Ia), (Ib) and (Ic), etc.) to form the glucoside and UDP.

The furofuran-type lignans which are glycosyl acceptor substrates include, for example, the lignans of structural formulae (Ia), (Ib), (Ic), (Id), (Ie), (If), (Ig) and (Ih), etc. Among others, the lignans of structural formulae (Ia), (Ib) and (Ic) are preferred, more preferably the lignans of structural formulae (Ia) and (Ic), and most preferably the lignan of structural formula (Ia).

### 3. Vector and Transformant Bearing the Same

In yet another embodiment, the present invention provides an expression vector comprising the polynucleotide of the present invention. The vector of the present invention comprises the polynucleotide of the present invention (e.g., any one of the polynucleotides (a) to (j) described above). Preferably, the expression vector of the present invention comprises any one of the polynucleotides (g) to (j) described above. More preferably, the expression vector of the present invention comprises a polynucleotide consisting of the nucleotide sequence represented by SEQ ID NO: 3 or 5, or a polynucleotide comprising a polynucleotide encoding a protein consisting of the amino acid sequence of SEQ ID NO: 4 or 6.

The vector of the present invention is generally constructed to contain an expression cassette comprising as the constituents (i) a promoter that can be transcribed in a host cell, (ii) the polynucleotide of the present invention linked to the promoter above (e.g., any one of the polynucleotides described in (a) to (j) above), and (iii) a signal that functions in a host cell with respect to the transcription termination and polyadenylation of RNA molecule. The vector thus constructed is introduced into a host cell. Methods for construction of the expression vector include those using a plasmid, phage, cosmid, etc. but are not particularly limited thereto.

Specific types of the vector are not particularly limited, and vectors capable of expressing in a host cell can be suitably chosen. That is, a suitable promoter sequence may be chosen depending upon the type of the host cell to reliably express the polynucleotide of the present invention, and a vector obtained by incorporating this sequence and the polynucleotide of the present invention into various plasmids or the like may be used as an expression vector.

The expression vector of the present invention contains an expression control region (for example, a promoter, a terminator, and/or a replication origin, etc.) depending on the type of a host to be introduced. A conventional promoter (for example, trc promoter, tac promoter, lac promoter, etc.) is used as a promoter for a bacterial expression vector. As a promoter for yeast, there are used, for example, a glyceraldehyde 3-phosphate dehydrogenase promoter, PH05 promoter, etc. As a promoter for fungi there are used, for example, amylase, trpC, etc. Additionally, a viral promoter (e.g., SV40 early promoter, SV40 late promoter, etc.) is used as a promoter for animal-derived host cell.

The expression vector preferably contains at least one selective marker. Such a marker that is available includes an auxotrophic marker (ura5, niaD), a drug-resistant marker (hygromycin, zeocin), a geneticin-resistant marker (G418r), a copper-resistant gene (CUP1) (Marin et al., Proc. Natl. Acad. Sci. USA, vol. 81, p. 337, 1984), a cerulenin resistant gene (fas2m, PDR4) (Junji Inokoshi et al., Biochemistry, vol. 64, p. 660, 1992; Hussain et al., Gene, vol. 101, p. 149, 1991, respectively), and the like.

The present invention provides a transformant in which the polynucleotide of the present invention (for example, the polynucleotide described in any one of (a) to (j) above) is introduced.

A method of preparing (method of producing) the transformant is not particularly limited and includes, for example, a method which comprises introducing the recombinant vector described above into a host followed by transformation. The host cell as used herein is not particularly limited and various known cells may be preferably used. Specific examples are bacteria such as Escherichia coli, etc., yeast (budding yeast Saccharomyces cerevisiae, fission yeast Schizosaccharomyces pombe), nematode (Caenorhabditis elegans), oocyte of African clawed frog (Xenopus laevis), etc. Culture media and conditions suitable for the host cells above are well known in the art. The organism to be transformed is not particularly limited, and includes various microorganisms, plants and animals given as examples of the host cells above.

For transformation of the host cell, there may be used generally known methods. For example, methods that can be used include, but not limited to, the electroporation method (Mackenxie D. A. et al., Appl. Environ. Microbiol., vol. 66, p. 4655-4661, 2000), the particle delivery method (method described in JPA 2005-287403 "Method of Breeding Lipid-Producing Fungus"), the spheroplast method (Proc. Natl. Acad. Sci. USA, vol. 75, p. 1929, 1978), the lithium acetate method (J. Bacteriology, vol. 153, p. 163, 1983), and methods described in Methods in yeast genetics, 2000 Edition: A Cold Spring Harbor Laboratory Course Manual, etc.

In another embodiment of the present invention, the transformant can be a plant transformant. The plant transformant in accordance with the embodiment can be acquired by introducing a recombinant vector bearing the polynucleotide in accordance with the present invention into a plant in such a manner that the polypeptide encoded by the said polynucleotide can be expressed.

Where a recombinant expression vector is used, the recombinant expression vector used to transform the plant is not particularly limited as far as the vector is capable of expressing the polynucleotide in accordance with the present invention in the said plant. Examples of such vectors include a vector bearing a promoter capable of constitutively expressing the polynucleotide in plant cells (e.g., a 35S promoter of cauliflower mosaic virus) in plant cells, and a vector inducibly activated by external stimulation.

Plants which are to be the target of transformation in the present invention may be any of entire plant bodies, plant organs (e.g., leaves, petals, stems, roots, seeds, etc.), plant tissues (e.g., epidermis, phloem, parenchyma, xylem, vascular bundles, palisade tissues, spongy tissues, etc.) or plant culture cells, or various types of plant cells (e.g., suspension culture cells), protoplasts, leaf slices, calli, and the like. Specific examples of plant species which are used for the transformation include, but are not limited to, those belonging to the Monocotyledoneae or the Dicotyledoneae.

In the introduction of genes into plants, conventional transformation methods known to those skilled in the art (e.g., the Agrobacterium method, gene gun, the PEG method, the electroporation method, etc.) are used. For example, the Agrobacterium-mediated method and the method of directly introducing into plant cells are well known. When the Agrobacterium method is used, the constructed plant expression vector is introduced into an appropriate Agrobacterium strain (e.g., Agrobacterium tumefaciens), followed by infection of aseptically cultured leaf discs with this strain according to the leaf disc method (Hirobumi Uchimiya, Manuals for Plant Gene Manipulation (I990), 27-31, Kodansha Scientific Co., Ltd., Tokyo). Thus, the transgenic plant can be obtained. In addition, the method of Nagel, et al. (Microbiol. Lett., 67, 325 (1990)) may also be used. This method involves introducing first, e.g., an expression vector into Agrobacterium and then introducing the transformed Agrobacterium into plant cells or plant tissues according to the method described in Plant Molecular Biology Manual (S. B. Gelvin, et. al., Academic Press Publishers). Herein, the "plant tissue" includes callus obtained by culturing plant cells. When the transformation is carried out using the Agrobacterium method, binary vectors (pBI121, pPZP202, etc.) can be used.

For direct transfer of genes into plant cells or plant tissues, the electroporation method and the gene gun method are known. When the gene gun is used, entire plant bodies, plant organs or plant tissues per se may be used, or may be used after preparation of protoplasts. The samples thus prepared can be bombarded using a gene transfer apparatus (e.g., PDS-1000 (BIO-RAD, Inc.), etc.). Bombardment conditions vary depending upon type of the plant or sample. Normally, the sample is bombarded under a pressure of about 450 to 2000 psi at a distance of about 4 to 12 cm.

The cells or plant tissues, in which the gene is introduced, are first selected by chemical resistance such as a hygromycin resistance, etc. and then regenerated into plant bodies in a conventional manner. Regeneration of plant bodies from the transformant cells can be performed by methods known to one skilled in the art, depending upon kind of plant cells.

When a plant culture cell is used as a host, transformation is performed by introducing the recombinant vector into the culture cell by means of gene gun, the electroporation method, etc. Calli, shoots, hairy roots or the like resulting from the transformation can be used for cell culture, tissue culture or organ culture as they are. Alternatively, these tissues or cells can be allowed to regenerate into a plant by any known conventional method for culturing plant tissues, which involves administering a plant hormone (auxin, cytokinin, gibberellin, abscisic acid, ethylene, brassinolide, etc.).

Whether or not the gene is introduced into a plant can be confirmed by PCR, Southern hybridization, northern hybridization or the like. For example, a DNA is prepared from transformed plants, DNA-specific primers are then designed, and PCR is subsequently performed. PCR can be performed under the same conditions as used for preparing the plasmids described above. Subsequently, the transformation can be confirmed by applying the amplified product to agarose gel electrophoresis, polyacrylamide gel electrophoresis, capillary electrophoresis or the like, staining the product with an appropriate dye, such as ethidium bromide, SYBR Green solution, etc. and then detecting the amplified product as a single band. Furthermore, the amplified products can also be detected by performing PCR using primers labeled with suitable labels, e.g., a fluorescent dye. Other methods that can also be employed herein involve binding the amplified product to a solid phase, such as a microplate, and then confirming the amplified product by fluorescence, an enzyme reaction or the like.

Once a transformed plant wherein the polynucleotide in accordance with the present invention is introduced into the genome is obtained, it is possible to acquire descendants from that plant body by sexual or vegetative propagation. Alternatively, the plant can be mass-produced from, for example, seeds, fruits, ears, tubers, tubercles, tubs, calli, protoplasts, etc., which are obtained from the plant or its descendants or their clones. Accordingly, the present invention also includes plant bodies in which the polynucleotide in accordance with the present invention is expressibly introduced, and descendants having the same properties as those of the plant bodies as well as tissues derived therefrom.

Methods for transformation of various plants have already been reported. Examples of the transgenic plants in accordance with the present invention include, but are not limited to, Forsythia koreana, Sesamum indicum, rice plant, tobacco, barley, wheat, rapeseed, potato, tomato, poplar, banana, eucalyptus, sweet potato, soy, alfalfa, lupinus, corn, cauliflower, rose, chrysanthemum, carnation, snapdragon, cyclamen, orchid, Eustome russellianum, freesia, gerbera, gladiolus, Grypsophila elegans, kalanchoe, lily, pelargonium graveolen, geranium, petunia, torenia, tulip, Arabidopsis thaliana, grapevine, Lotus japonicus, and the like.

### 4. Method of Producing Protein of the Invention

In yet another embodiment, the present invention provides a method of producing the protein of the present invention using the transformants described above.

Specifically, the protein of the present invention may be obtained by isolating and purifying the protein of the present invention from the culture of the transformants described above. As used herein, the culture is intended to mean any of culture broth, cultured bacteria or cultured cells, and the homogenate of cultured bacteria or cultured cells. Conventional methods may be used to isolate and purify the protein of the present invention.

Specifically, when the protein of the present invention accumulates within cultured bacteria or within cultured cells, a crude extract of the protein of the present invention may be obtained by culturing the bacteria or cells, then disrupting the bacterial or cells using a conventional technique (e.g., ultrasonication, lysozymes, freezing and thawing, etc.) and applying a conventional method (e.g., centrifugation or filtration, etc.). When the protein of the present invention is accumulated in culture broth, the culture supernatant containing the protein of the present invention can be obtained, after completion of the incubation, by separating the bacteria or cells from the culture supernatant in a conventional manner (e.g., centrifugation, filtration, etc.).

Purification of the protein of the present invention contained in the extract or culture supernatant obtained as described above can be performed by a conventional method of separation and purification. The separation and purification methods including, e.g., ammonium sulfate precipitation, gel filtration chromatography, ion exchange chromatography, affinity chromatography, reversed phase high performance liquid chromatography, dialysis, ultrafiltration, etc. may be used singly or in a suitable combination.

### 5. Method of Producing Glucoside

The present invention further provides a method of producing a glucoside using the protein of the present invention.

The protein of the present invention catalyzes the reaction of transferring glucose from the glycosyl donor UDP-glucose to the glycosyl acceptor substrate furofuran-type lignan (in detail, which is the reaction of transferring glucose to the hydroxy group at the position 4 and/or position 4' of furofuran-type lignans). Accordingly, the glucoside can be produced from the glycosyl acceptor substrate and the glycosyl donor as the starting materials by using the protein of the present invention. The glycosyl acceptor substrate includes various furofuran-type lignans such as those of structural formula (Ia), (Ib), (Ic), (Id), (Ie), (If), (Ig), (Ih), etc. Among others, the lignans of structural formulae (Ia), (Ib) and (Ic) are preferred, more preferably the lignans of structural formulae (Ia) and (Ic), and most preferably the lignan of structural formula (Ia).

The glucoside can be produced, for example, by preparing a solution containing 1 mM of the glycosyl acceptor substrate, 2 mM of the glycosyl donor, 50 mM of calcium phosphate buffer (pH 7.5) and 20 µM of the protein of the present invention and reacting them at 30°C for 30 minutes. The glucoside can be isolated/purified from the solution by known methods. Specifically, e.g., ammonium sulfate precipitation, gel filtration chromatography, ion exchange chromatography, affinity chromatography, reversed phase high performance liquid chromatography, dialysis, ultrafiltration, etc. can be used alone or in an appropriate combination.

Hereinafter, the present invention is described more specifically with reference to EXAMPLES below but is not deemed to be limited thereto.

### EXAMPLE 1

### Gene Cloning

The molecular biological techniques used in this EXAMPLE were performed according to the methods described in Molecular Cloning (Sambrookra, Cold Spring Harbour Laboratory Press, 2001), unless otherwise described in detail.

Total RNA was extracted from the leaves of Forsythia koreana of the family Oleaceae using an RNeasy Plant Mini kit (QIAGEN). Subsequently, PolyA(+) RNA was isolated using an Oligotex-dT mRNA kit (TaKaRa Bio). Using 4 µg of this poly(+) RNA, a cDNA library was prepared using a lambda ZAP II directional cDNA library synthesis kit (Stratagen) by the method as recommended by the manufacturer.

The above cDNA library of approximately 500,000 pfu was screened by plaque hybridization using the UGT71C1 gene of Arabidopsis thaliana (GenBank Accession No.: NM_12852) as a screening probe. The probe was labeled by PCR with a non-radioactive isotope DIG-Nucleic Acid detection system (Roche) under the conditions as recommended by the manufacturer. In this case, the PCR solution (50 µl) was composed of cDNA derived from the root of Arabidopsis thaliana, 1x Taq buffer (TakaRa Bio), 0.2 mM dNTPs, 0.2 pmol each/µl of gene-specific primers (SEQ ID NOs: 1 and 2) and 1.25 U Taq polymerase. PCR was performed, after reacting at 94°C for 5 minutes, by repeating 30 cycles in total, each cycle consisting of 94°C for 1 minute, 52°C for 1 minute and 72°C for 2 minutes, and finally treating at 72°C for 5 minutes. The PCR product that the primer and unreacted dNTP were removed through a Mini QuickSpin column (Roche) was used as the probe.
AtUGT71C1-Fw:
   5'-ATGGGGAAGCAAGAAGATGCAGAG-3' (SEQ ID NO: 1)
AtUGT71C1-Rv:
   5'-CTACTTACTTATAGAAACGCCGT-3' (SEQ ID NO: 2)

Screening of the library and detection of positive clones were conducted by the method as recommended by the manufacturer, using a non-radioisotope DIG-Nucleic Acid detection system (Roche Diagnostics). Hybridization was performed in 5x SSC containing 30% formamide at 37°C overnight, and the membrane was washed in 4x SSC and 1% SDS at 55°C for 20 minutes. The positive clones obtained by screening of approximately 400,000 plaques were sequenced on a DNA Sequencer Model 3100 (Applied Biosystems) by primer walking using synthetic oligonucleotide primers to obtain cDNA sequences. The cDNA sequences obtained were subjected to homology analysis with Blastx program (http://blast.ncbi.nlm.nih.gov/Blast.cgi) to obtain Forsythia koreana UGT-like genes (RengUGT).

Clone 13 (RengUGT13) shown in SEQ ID NOs: 3 and 4 and clone 14 (RengUGT14) shown in SEQ ID NOs: 5 and 6 had 90% sequence homology to each other on an amino acid level, and showed approximately 40% homology to UGT71C1 of Arabidopsis thaliana used as the probe for the Blastx analysis and approximately 56% homology to Sesamum indicum UGT71A9 (glycosyltransferase specific for the position 2 of the lignan of structural formula (III)), but no clear function could be inferred. Therefore, the clones were expressed in Escherichia coli to analyze their function.
cDNA Sequence of RengUGT13: 
ATGGCAGAAACAAAGAAATCAGAGCTTGTTTTCATTCCTGTTCCAGGTGT
AGGTCACTTGATATCAACTATTGAGATGGCTGAGCTTCTCACTGATAGAG
ATGAACGCCTCTCGATCACAGTCATCATCATGAAGTTGCCAATGGAGTCA
AAAACCGATTTCTATTCCCGAAAATCCAATTCGCGTATACGTTTCATAGA
ATTTTCTCTCAACCAGCCCATTACACCCAACAACTTTGTCACTCATTTCAT
CGAAAGCCACAAGGATCCTATAAGAGATGCTGTCACGAAAATAGTTCGT
GATGAGTCCAACTCGATCAGACTTGCTGGATTCGTTATTGATATGTTTTGT
ACTACTATGATTGACGTAGCCAATGAGTTTGGTGTCCCGACTTATGTTTTC
TTTACAACTACTGCTGCACTGCTTGGGTTCACCTTCTATTTGCAGAGTCGC
AGCGATGAACAGAAGTTGGATGTGACAGAATACAAGAACTCTAATGCTG
AGTTATTGATTCCCACGTACATAAATCCAGTTCCTGCTAACGTATTTCCTT
CGAGATTCTTTGATAAGGATGGTTTGGCTATGTTCCTCGGTATGGCAAGA
AGGTTTAGAGAGACCAAGGGAATTATGATTAACACCTTCTTGGATTTAGA
AGCTCATGCAATGAAGTCCCTCTCAGATGATCATACAATCCCACCGGTCT
ACTCAATAGGCCCAATAATTCATGTTACGGCTGAAAATGATGACGACAA
CAAAGATTATGACGAGATCATCAAGTGGCTTCACGAACAACCTGTTTCTT
CTGTTGTTTTTCTTTGCTTTGGAAGTATGGGATTTTTTGATGATGAACAGG
TTAAAGAGATTGCGGTTGCACTTGAAAAGAGTGGCCACCGGTTCTTGTGG
TCATTGAGAAAGCCTCCTCCCAAAGACAGGTTCGAGTATCCATCAGACTA
CGAAAATCTTGAAGAAATTTTGCCAGAAGGGTTCTTGCAACGTACTGCAG
GAATCGGGAAGGTGATTGGATGGGCTCCACAAGTGGCCGTCTTATCTCAC
CACTCGGTGGGAGGCTTTGTGTCGCATTGTGGTTGGAATTCGACACTGGA
AAGTGTCTGGTGTGGAGTGCCAATAGCAGCATGGCCAATGTATGCTGAG
CAGCAGACTAATGCCTTCGAGTTGGTGAAGGACTTGGGAATTGCTGTGGA
GATTAAGATGGACTATAGGAGGGGCAGTGATGTGATTGTGAAGGCTGAA
GAAATTGAGAAGGGAATCAGGCACCTAATGGAGCCTGATAGTGAAATGA
GGAATAAGATGAAACAGATGAAAAACAAGAGTAGATTGGCTTTGATGGA
AGGTGGCTCTTCCTACGATTTCTTGAGACATTTCATCGATAACATTCCAAT
GACGGAT (SEQ ID NO: 3)
Amino Acid Sequence of RengUGT13: 
MAETKKSELVFIPVPGVGHLISTIEMAELLTDRDERLSITVIIMKLPMESKTDF
YSRKSNSRIRFIEFSLNQPITPNNFVTHFIESHKDPIRDAVTKIVRDESNSIRLAG
FVIDMFCTTMIDVANEFGVPTYVFFTTTAALLGFTFYLQSRSDEQKLDVTEY
KNSNAELLIPTYINPVPANVFPSRFFDKDGLAMFLGMARRFRETKGIMINTFL
DLEAHAMKSLSDDHTIPPVYSIGPIIHVTAENDDDNKDYDEIIKWLHEQPVSS
VVFLCFGSMGFFDDEQVKEIAVALEKSGHRFLWSLRKPPPKDRFEYPSDYEN
LEEILPEGFLQRTAGIGKVIGWAPQVAVLSHHSVGGFVSHCGWNSTLESVWC
GVPIAAWPMYAEQQTNAFELVKDLGIAVEIKMDYRRGSDVIVKAEEIEKGIR
HLMEPDSEMRNKMKQMKNKSRLALMEGGSSYDFLRHFIDNIPMTD (SEQ ID
NO: 4)
cDNA Sequence of RengUGT1: 
ATGGCAGAAACAAAGAAATCAGAGCTTGTTTTCATTCCTGCACCAGGAAT
AGGTCACTTGATATCAACTATTGAGTTGGCTAAGCTTCTCACTGATAGAG
ATGAGCACCTCTCGATCACAGTCCTCATCTTGAAGTTGCCAATGGAGTCG
AAAACCGATTCCTATTCCCAAAAATCCAATTCGCGTATACGTTTCATAGA
ATTATCTCTCAATCAACCTATTACACCCAACAACTTTGTCACTGATTTCAT
CGAAGGCCACAAGGATCCTATAAGAGATGCTGTCACGAAAATAGTTCGT
GATGAGTCCAACTCTATTAGACTTGCTGGATTCGTTATTGATATGTTTTGT
ACTACTATGATTGATGTAGCCAATGAGTTTGGTGTCCCGACGTATGTTTTC
TTTACAACTACTGCTGCAATGCTTGGGTTCATCTTCTATTTGCAGAGTCGC
GGCGATGAACAGAAGTTGGATGTGACTGAGTACAAGAACTCAAATACTA
AGTTATTGATTCCCACGTACATAAATCCGGTTCCTGCTAATGTATTTCCTT
CTAAATTATTTGATAAGGATAGTTTGGCTCCATTCGTCAGTATGGCAAGA
AGGTTTAGAGAGACCAAGGGAATTTTGATTAACACCTTCTTGGATTTAGA
AGCTTATGCATTGAAGTCCCTCTCTGATGATCATACAATCCCACCGGTCT
ACTCAATAGGCCCGATACTTCATGTTAAGGTTGAAAATGATGACAAAAA
GAAAGATTATGACGAGATCATCAATTGGCTTCACGAACAACCTGTTTCGT
CTGTTGTTTTTCTTTGCTTTGGAAGTTTGGGTTGTTTTGATGTCGAACAGG
TTAAAGAGATTGCGGTTGCACTTGAAAAGAGTGGCCACCGGTTCTTGTGG
TCATTGAGAAAGCCTCCTCCCAAAGACTTCGAGCATCCATCAGACTACGA
AAATTTTGAAGAAGTATTGCCAGAAGGGTTCTTGCAACGTACAGCAGGA
ATCGGGAAGGTGATTGGATGGGCGCCACAAGTGGCCGTCTTATCTCACCA
TTCGGTGGGAGGCTTTGTGTCGCATTGTGGTTGGAATTCGACGCTGGAAA
 
GTGTCTGGTGTGGAGTGCCAATAGCGGCATGGCCAATGTATGCCGAACA
GCAGACTAATGCCTTTGAGTTGGTGAAGGACTTGGGAATTGCTGTGGAGA
TTAAGATGGACTATAGGAAGGGCAGTGATGTGATTGTGAAGGCTGAAGA
AATTGAGAAGGGAATCAAGCACCTAATGGAGCCTGATAGTGAAATGAGG
AATAAGATGAAACAGATGAAAAGCAAGAGTAGATTGGCTTTGATGGAAG
GTGGCTCTTCTTACAATTTCTTGAGGCGTTTCATCGATAACATTCCAATGA
CGGAT (SEQ ID NO: 5)
Amino Acid Sequence of RengUGT14:

MAETKKSELVFIPAPGIGHLISTIELAKLLTDRDEHLSITVLILKLPMESKTDSY
SQKSNSRIRFIELSLNQPITPNNFVTDFIEGHKDPIRDAVTKIVRDESNSIRLAG
FVIDMFCTTMIDVANEFGVPTYVFFTTTAAMLGFIFYLQSRGDEQKLDVTEY
KNSNTKLLIPTYINPVPANVFPSKLFDKDSLAPFVSMARRFRETKGILINTFLD
LEAYALKSLSDDHTIPPVYSIGPILHVKVENDDKKKDYDEIINWLHEQPVSSV
VFLCFGSLGCFDVEQVKEIAVALEKSGHRFLWSLRKPPPKDFEHPSDYENFEE
VLPEGFLQRTAGIGKVIGWAPQVAVLSHHSVGGFVSHCGWNSTLESVWCGV
PIAAWPMYAEQQTNAFELVKDLGIAVEIKMDYRKGSDVIVKAEEIEKGIKHL
MEPDSEMRNKMKQMKSKSRLALMEGGSSYNFLRRFIDNIPMTD (SEQ ID
NO: 6)

### EXAMPLE 2

### Construction of Expression Vector

The full length ORFs of RengUGT13 and RengUGT14 were amplified by PCR using the primers with added restriction enzyme sites (SEQ ID NOs: 7 and 8), which are given below.

This set of primers share a common sequence, which permits to amplify the two RengUGT clones. The underlined nucleotide sequences in the primers are the restriction enzyme recognition sequences added to the primers.
CACC-NdeI-RengUGT-Fw:
   5'-CACCCATATGGCAGAAACAAAGAAATCAGA -3' (SEQ ID NO: 7)
BglII-RengUGT-Rv:
   5'-AGATCTTTAATCCGTCATTGGAATGTTAT -3' (SEQ ID NO: 8)

The PCR solution (50 µl) was composed of1 µl of template DNA (phage solution after secondary screening), 1x ExTaq buffer (TaKaRa Bio), 0.2 mM dNTPs, 0.4 pmol each/µl of the primers and 2.5U of ExTaq polymerase. PCR was performed, after reacting at 94°C for 3 minutes, by repeating total 30 cycles for amplification, each cycle consisting of 94°C for 1 minute, 50°C for 1 minute and 72°C for 2 minutes. The PCR products were electrophoresed on a 1% agarose gel and stained with ethidium bromide to give the amplified band at about 1.4 kb predicted from each template DNA.

These PCR products were subcloned into pENTR-TOPO Directional vector (Invitrogen) according to the method as recommended by the manufacturer. The clones were sequenced on a DNA Sequencer Model 3100 (Applied Biosystems) by primer walking using synthetic oligonucleotide primers to verify that any mutation by PCR was not revealed in the inserted fragments.

Approximately 1.4 kb fragments of RengUGT13 and RengUGT14 were excised using the restriction enzyme sites ofNdeI and BglII added to the primers and ligated to the NdeI and BglII site of Escherichia coli expression vector pET15b (Novagen) to give the Escherichia coli expression vector of this enzyme gene. The open reading frame of His tag upstream of the NdeI site of this vector was matched with that of the two RengUGT genes; a design was intended to express a chimeric protein of the two RengUGTs fused to the His tag.

### EXAMPLE 3

### Analysis of Enzyme Function

To clarify the biochemical functions of the enzymes, the two enzymes were expressed in Escherichia coli. Using the plasmid for two RengUGT Escherichia coli expression obtained above, Escherichia coli BL21 (DE3) strain was transformed in a conventional manner. The resulting transformants were shake cultured in 4 ml of LB medium (10 g/l tryptone peptone, 5 g/l yeast extract, 1 g/l NaCl) containing 50 µg/ml of ampicillin at 37°C overnight. When the cells reached the stationary phase, 4 ml of the culture broth was inoculated onto 80 ml of fresh medium of the same composition, followed by shake culture at 37°C. At the point when the cell turbidity (OD 600) reached approximately 0.5, IPTG in a final concentration of 0.5 mM was added to the cells, followed by shake culture at 18°C for 20 hours.

The following procedures were all carried out at 4°C. The transformants cultured were collected by centrifugation (5,000 x g, 10 mins.) and suspended by adding 1 ml/g cell of Buffer S (20 mM HEPES buffer (pH 7.5), 20 mM imidazole and 14 mM β-mercaptoethanol). Subsequently, the suspension was ultrasonicated (15 secs x 8 times) and then centrifuged (15,000 x g, 15 mins.). The supernatant obtained was recovered as a crude enzyme solution. The crude enzyme solution was loaded onto His SpinTrap (GE Healthcare), which had been equilibrated with Buffer S, and centrifuged (70 x g, 30 secs.). After washing with the buffer, the proteins bound to the column were eluted stepwise with 5 ml each of Buffer S containing 100 mM and 500 mM imidazole. In each of the eluted fractions, the buffer was replaced through a Microcon YM-30 (Amicon) by 20 mM HEPES buffer (pH 7.5)/14 mM β-mercaptoethanol (magnification of dialysis, x1000).

As a result of the SDS-PAGE analysis, in the fraction eluted with 200 mM imidazole, the protein was confirmed at approximately 50 KDa of the estimated molecular weight for the two RengUGTs. This fraction was used for the enzyme analysis (FIG. 2).

Conditions for the standard enzyme reaction are as follows. The reaction solution (1 mM UDP-glucose, 100 µM glycosyl acceptor substrate, 100 mM potassium phosphate buffer (pH 7.5) and 25 µl of purified enzyme solution) was prepared into a 50 µl solution with distilled water and reacted at 30°C for an hour. The reaction was stopped by adding 100% acetonitrile containing 50 µl of 0.1% trifluoroacetic acid (TFA), followed by analysis on reversed phase HPLC.

Lc-2010c (Shimadzu Corp.) was used for LC; eluent A: 0.1% TFA and eluent B: 0.1% TFA/90% acetonitrile as the moving phase; Develosil-C30-UG5 (Nomura Chemical, 4.6 mm x 150 mm) as the column at 40°C. After the column was equilibrated, elution was performed at a flow rate of 1 ml/min in a linear density gradient of B5% → B100% for 20 minutes and B100% for 5 minutes. Detection was performed by measuring the absorption at 280 nm. Based on the analysis that the spectra from 220 nm to 330 nm were measured using SPD-10AV (Shimadzu Corp.), search was made for substances having two absorption maxima (230 nm and 280 nm) inherent to lignans. Under the conditions, the lignan of structural formula (Ia) was detected at approximately 11.6 minutes, the lignan of structural formula (Ib) at approximately 12.2 minutes, the lignan of structural formula (Ic) at approximately 13.8 minutes, and the lignan of structural formula (V) at approximately 9.8 minutes.

The reaction solution of the lignan of structural formula (Ia) and RengUGT13 or RengUGT14 was analyzed for HPLC under the conditions described above. As a result, the peak eluted at about 8.7 minutes was newly detected (FIG. 3; marked with asterisk). The peak is dependent on glycosyl donor UDP-glucose and found to be the peak of the lignan glycoside in which glucose is added to the lignan of structural formula (Ia).

Subsequently, the peak was fractionated and provided for MS analysis under the conditions described below.

For LC-MS, a Develosil C30-UG-3 column (Nomura Chemical, 3.0 mm x 150 mm) was used in a LCMS-IT-TOF system (Shimadzu Corp.); the moving phase used was water containing 0.1% formic acid as eluent A and 100% acetonitrile containing 0.1 % formic acid as eluent B. Elution was performed in a linear density gradient (eluent B 5% → 100%) for 20 minutes, followed by isocratic elution with eluent B 100% for 5 minutes (flow rate: 0.2 ml/min, column oven: 40°C). Detection was performed using a photodiode array detector (SPD-M10A, Shimadzu Corp.) to collect data at 230 to 500 nm and measure the chromatogram at A280nm. The measurement condition of MS was the measurement in a negative mode (ionization: ESI, interface voltage: -3.5kV).

Under the conditions, the peak was assumed to be a monoglucoside which gave a molecular ion of 519.16391 [M-H]- (FIG. 4). From this it was shown that RengUGT13 and RengUGT4 were enzymes having the activity of transferring one molecule of glucose to the lignan of structural formula (Ia).

The expression level of RengUGT14 recombinant protein was high in Escherichia coli (FIG. 2), and the glycosyl acceptor selectivity was evaluated using this enzyme. When the activity against the lignan of structural formula (Ia) is made 100, the transglycosylation activity as high as 112 was found against the lignan of structural formula (Ib) which is the same furofuran-type lignan and the transglycosylation activity as high as 128 against the lignan of structural formula (Ic). On the other hand, little activity was shown against the lignan of structural formula (V), which is a non-furofuran-type lignan. The foregoing results revealed that RengUGT4 is a glycosyltransferase specific to the position 4 of furofuran-type lignans.

### EXAMPLE 4

### Gene Expression Analysis

In order to identify the functional domains of RengUGT13 and RengUGT4 genes, the expression profile of the gene from Forsythia koreana in each organ was analyzed by RT-PCR in a similar manner to the method as described in "Ono, E., et al. (2006) Proc. Natl. Acad. Sci. USA 103, 10116-10121."

Total RNA was extracted from each organ of Forsythia koreana (leaf, young bud, petal and culture cell (derived from callus)) in a manner similar to EXAMPLE 1, and 0.5 µg of the total RNA was subjected to reverse transcription (RT) using a Random Oligo-dT primer to acquire cDNA derived from each organ. The cDNA was used as a template for PCR.

The gene-specific primers (SEQ ID NOs: 9 to 12) used for RT-PCR were designed to be able to distinguish between the RengUGT13 and RengUGT4 genes. Forsythia koreana ribosomal RNA (Forsythia koreana rRNA; GenBank Accession No.: AF534808) was taken as an internal standard gene and amplified using the following gene-specific primers (SEQ ID NOs: 13 and 14).

### <Primers for RengUGT13 gene>

Reng13-RT-FW2:
   5'-TAGCGGATCAACCAACTAAAC -3' (SEQ ID NO: 9)
Reng13-RT-RV:
   5'-TCTTGCCATACCGAGGAACAT -3' (SEQ ID NO: 10)

### < Primers for RengUGT14 gene>

Reng14-RT-FW2:
   5'-TAGCAGATCAACCCAGTAAAT -3' (SEQ ID NO: 11)
Reng14-RT-RV:
   5'-TCTTGCCATACTGACGAATGG -3' (SEQ ID NO: 12)

### <Primers for Forsythia koreana rRNA>

FsrRNA-Fw:
   5'- TAAAACGACTCTCGGCAACGGA -3' (SEQ ID NO: 13)
FsrRNA-Rv:
   5'- ATCCCGCCTGACCTGGGGTCGCT -3' (SEQ ID NO: 14)

The PCR solution (50 µl) was composed of 1 µl of template cDNA, 1x ExTaq buffer (TaKaRa Bio), 0.2 mM dNTPs, 0.4 pmol each/µl of the primers and 2.5U ExTaq polymerase. PCR was performed, after reacting at 94°C for 3 minutes, by repeating total 20 to 35 cycles for amplification, each cycle consisting of 94°C for 1 minute, 50°C for 1 minute and 72°C for 2 minutes, and the conditions that gene expression was not saturated were set up for each gene.

The PCR product was electrophoresed on 1% agarose and stained with ethidium bromide for detection (FIG. 5). As a result, the RengUGT13 and RengUGT14 genes were expressed most highly in the leaves. The expression was recognized also in the buds and petals. However, the RengUGT14 gene was expressed specifically in the culture cells; it was therefore considered that this enzyme was responsible for the transfer of sugar to the lignan in the culture cells.

The foregoing results indicate that RengUGT13 and RengUGT14 are the enzymes, which are similar in structure, function and expression domain.

### INDUSTRIAL APPLICABILITY

The genes for the enzymes which catalyze the transfer of glucose to the position 4 and/or position 4' of furofuran-type lignans could be identified. By using the enzymes, glucose can be artificially transferred in vitro specifically to the position 4 and/or position 4' of furofuran-type lignans. Accordingly, the enzymes can contribute to the development of new functional food materials, molecular breeding of secondary metabolism, etc.

Therefore, the present invention can be used over a wide range, including agriculture, food industry, drug industry and other industries related thereto and is extremely beneficial in this regard.

### [Sequence Listing Free Text]

SEQ ID NO: 1: synthetic DNA
SEQ ID NO: 2: synthetic DNA
SEQ ID NO: 7: synthetic DNA
SEQ ID NO: 8: synthetic DNA
SEQ ID NO: 9: synthetic DNA
SEQ ID NO: 10: synthetic DNA
SEQ ID NO: 11: synthetic DNA
SEQ ID NO: 12: synthetic DNA
SEQ ID NO: 13: synthetic DNA
SEQ ID NO: 14: synthetic DNA

## Claims

1. A polynucleotide of any one of (a) to (f) below:
(a) a polynucleotide comprising a polynucleotide consisting of the nucleotide sequence represented by SEQ ID NO: 3 or 5;
(b) a polynucleotide comprising a polynucleotide encoding a protein consisting of the amino acid sequence represented by SEQ ID NO: 4 or 6;
(c) a polynucleotide comprising a polynucleotide encoding a protein consisting of the amino acid sequence wherein 1 to 15 amino acids are deleted, substituted, inserted and/or added in the amino acid sequence represented by SEQ ID NO: 4 or 6, and having a glucosyltransferase activity against a lignan;
(d) a polynucleotide comprising a polynucleotide encoding a protein that has an amino acid sequence having a homology of at least 80% to the amino acid sequence represented by SEQ ID NO: 4 or 6, and has a glucosyltransferase activity against a lignan;
(e) a polynucleotide comprising a polynucleotide encoding a protein that hybridizes under stringent conditions with a polynucleotide consisting of a nucleotide sequence complementary to the nucleotide sequence represented by SEQ ID NO: 3 or 5, and has a glucosyltransferase activity against a lignan; or,
(f) a polynucleotide comprising a polynucleotide encoding a protein that hybridizes under stringent conditions with a polynucleotide consisting of a nucleotide sequence complementary to the nucleotide sequence of a polynucleotide encoding a protein consisting of the amino acid sequence represented by SEQ ID NO: 4 or 6, and has a glucosyltransferase activity against a lignan.

2. The polynucleotide according to claim 1, which is any one of (g) to (j) below:
(g) a polynucleotide comprising a polynucleotide encoding a protein consisting of the amino acid sequence wherein not exceeding 10 amino acids are deleted, substituted, inserted and/or added in the amino acid sequence represented by SEQ ID NO: 4 or 6, and having a glucosyltransferase activity against a lignan;
(h) a polynucleotide comprising a polynucleotide encoding a protein that has an amino acid sequence having a homology of at least 90% to the amino acid sequence represented by SEQ ID NO: 4 or 6, and has a glucosyltransferase activity against a lignan;
(i) a polynucleotide comprising a polynucleotide encoding a protein that hybridizes under high stringent conditions with a polynucleotide consisting of a nucleotide sequence complementary to the nucleotide sequence represented by SEQ ID NO: 3 or 5, and has a glucosyltransferase activity against a lignan; or,
(j) a polynucleotide comprising a polynucleotide encoding a protein that hybridizes under high stringent conditions with a polynucleotide consisting of a nucleotide sequence complementary to the nucleotide sequence of a polynucleotide encoding a protein consisting of the amino acid sequence represented by SEQ ID NO: 4 or 6, and has a glucosyltransferase activity against a lignan.

3. The polynucleotide according to claim 1 or 2, wherein the glucosyltransferase activity against a lignan is a glucosyltransferase activity against a furofuran-type lignan.

4. The polynucleotide according to claim 3, wherein the glucosyltransferase activity against a furofuran-type lignan is a glucosyltransferase activity against the hydroxy group at position 4 and/or position 4' of a furofuran-type lignan.

5. The polynucleotide according to claim 3 or 4, wherein the furofuran-type lignan is a lignan represented by structural formula (Ia), (Ib) or (Ic) below.

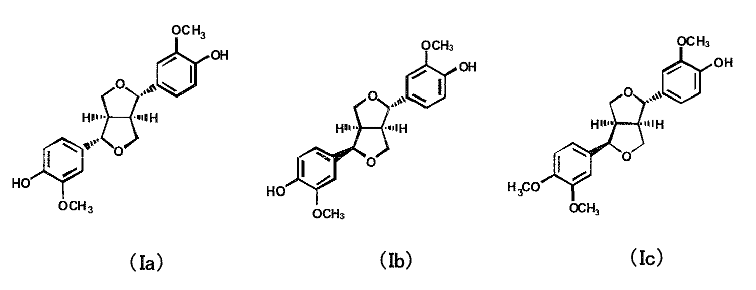

6. The polynucleotide according to claim 1, which comprises a polynucleotide consisting of the nucleotide sequence represented by SEQ ID NO: 3 or 5.

7. The polynucleotide according to claim 1, which comprises a polynucleotide encoding a protein consisting of the amino acid sequence represented by SEQ ID NO: 4 or 6.

8. The polynucleotide according to any one of claims 1 to 7, which is a DNA.

9. A protein encoded by the polynucleotide according to any one of claims 1 to 8.

10. A vector comprising the polynucleotide according to any one of claims 1 to 8.

11. A transformant, wherein the polynucleotide according to any one of claims 1 to 8 is introduced.

12. A transformant, wherein the vector according to claim 10 is introduced.

13. A method for producing the protein according to claim 9, which comprises using the transformant according to claim 11 or 12.

14. A method for producing a glucoside, which comprises forming the glucoside from UDP-glucose and a glycosyl acceptor substrate using the protein according to claim 9 as a catalyst.

15. The method according to claim 14, wherein the glycosyl acceptor substrate is a furofuran-type lignan.

16. The method according to claim 15, wherein the furofuran-type lignan is a lignan represented by structural formula (Ia), (Ib) or (Ic) below.
